# EUROPEAN PATENT SPECIFICATION

(11) **EP 0 242 033 B1**
(45) Date of publication and mention of the grant of the patent: **26.01.1994**
(21) Application number: 87301806.3
(22) Date of filing: 02.03.1987
(51) Int. Cl.: C07C 13/44, C07C 69/76, C07D 207/38, C07D 207/44, C07F 7/08, C08G 61/00, C08G 61/12

(54) **Compositions comprising arylcyclobutene and polymeric compositions prepared therefrom**
Arylcyclobuten enthaltende Zusammenstellungen und polymerische Zusammenstellungen, die daraus hergestellt werden
Compositions contenant de l'arylcyclobutène et compositions polymères obtenues à partir de celles-ci

(30) Priority: 28.02.1986 US 835013; 09.06.1986 US 872372
(43) Date of publication of application: 21.10.1987
(73) Proprietor: THE DOW CHEMICAL COMPANY, Midland Michigan 48640-1967 (US)
(72) Inventor: Kirchhoff, Robert A., Midland Michigan 48640 (US); Schrock, Alan K., Midland Michigan 48640 (US); Hahn, Stephen F., Midland Michigan 48640 (US)
(74) Representative: Burford, Anthony Frederick

(56) References cited:
- EP-A- 0 227 124
- EP-A- 0 229 757
- EP-A- 0 235 128
- WO-A-85/05355
- WO-A-86/01503
- US-A- 4 540 763
- TETRAHEDRON LETTERS, no. 22, 1977, pages 1863-1866; J.M. RIEMANN et al.: " Formation of methyl-substituted 2-indanones and 1,2-dihydrobenzocyclobutenes by the pyrolysis of o-, m-, and p-tolyl propargyl ethers"
- TETRAHEDRON LETTERS, no. 22, 1977, pages 1867-1870; J.M. RIEMANN et al.: "Formation of cyclobuta(b)pyridine and cyclobuta(c)pyridine by the pyrolysis of propargyl 4-pyridyl ether"

## Description

The present invention relates to compositions comprising a reactive arylcyclobutene moiety and a molecular group bonded to the aryl group of the arylacyclobutene moiety and to polymers prepared from such compositions.

Thermoset resins are compositions which can solidify irreversibly upon heating. Such resins are useful in many engineering applications, such as for example, as coatings, structural laminates, adhesives, films, composites and the like. Examples of conventional forming techniques are transfer molding, compression molding, and hand lay-up processing.

Desirable thermoset resins possess chemical resistance, tensile strength, temperature resistance, electro-insulative or electro-conductive properties and other properties which encourage their use as engineering materials. Such properties depend on the chemical structure of the resin or materials added to the resin. For example, resins comprised of aromatic structures, especially aromatic polyamides and polyimides intrinsically possess thermal and oxidative stability. Unfortunately, the preparation and curing of such resins require the handling of highly toxic and volatile compositions. Furthermore, the resins are difficult to form in molding processes, and are at times undesirably insoluble in many organic solvents.

Another class of engineering polymeric compositions are thermoplastic polymeric compositions. Such compositions soften upon heating and can be formed into many useful shapes. Upon cooling, the compositions harden to the desired shapes. Advantageously, the polymeric compositions exhibit chemical resistance and are structurally durable. Examples of suitable thermoplastic polymeric compositions are those prepared from polycarbonate thermoplastic compositions.

In recent years the search for high performance materials, especially high temperature-resistant polymers, has gained momentum. In order for a material to have stability at high temperatures, it must fulfill several requirements including a high melting or softening temperature, a high modulus or rigidity, a resistance to solvent and chemical degradation, and toughness. The intrinsic thermal and oxidative stability of aromatic structures has long been recognized, and a variety of polymers have been made in which benzene rings are linked together by various connecting groups. Among the more stable aromatic polymers that fulfill the requirements of high temperature resistance are the polybenzimidazoles, the polybenzoxazoles and the polyimides. Of these polymers, the polyimides have had the most interest.

The major difficulty encountered in the commercial development of these materials is that they are usually obtained in the form of a powder which cannot be readily fabricated into useful objects.

The polyimides prepared from aliphatic diamines and aromatic carboxylic acids are generally soluble and thermoplastic. Aliphatic polyimides have been prepared from bis(dienophiles) and a diene such as cyclopentadiene. Such reactions often involve gas evolution.

Aromatic polyimides, such as polypyromellitimides, have a spectrum of superior properties. Those polyimides may be prepared by the reaction of an aromatic dianhydride with an aromatic diamine to give a soluble polyamic acid, which on cyclodehydration gives the insoluble desired product.

High performance plastics reduce the weight of mechanical components, and not just by virtue of their densities. Their high performance properties allow greater design stresses, and often elements can be downsized accordingly. In recent years, aromatic polyimides have become widely accepted as premium, high performance engineering plastics. These resins are well-known for having excellent properties at elevated temperatures (i.e., chemical resistance) but are also costly. Historically, polyimide resins are difficult to fabricate into objects other than fibers and films. The most common methods of manufacturing parts having the highest strength and temperature properties are hot compression-molding, machining from hot-compression molded or extruded rod, and direct forming (a process similar to the powder-metallurgy processes). Given the synthetic and fabrication difficulties, a new route to polyimides is desirable.

A further problem with the preparation of certain polyimides is the need for the use of catalysts, initiators or curing agents. The presence of such compounds often results in the preparation of impure polymeric compositions. Further, the presence of such compounds often results in undesirable properties in such polymeric compositions. What are needed are monomers which prepare polyimides wherein the polymers can be easily processed, for example, fabricated into useful objects. What are further needed are monomers which can be polymerized in a manner such that no gas is evolved. What are further needed are monomers which can be polymerized without the need for catalysts, curing agents or initiators.

In US-A-4,540,763, poly(arylcyclobutene) polymers are disclosed. Such polymers are prepared from monomers which contain at least 2 arylcyclobutene moieties per monomer. The polymers exhibit excellent mechanical and physical properties such as high strength, thermal stability and glass transition temperatures. Unfortunately, because of the thermosetting nature of the polymers, the polymers are difficult to process in compression molding processes.

It would be desirable to have a new class of monomeric compounds which are suitable for providing thermoset and/or thermoplastic engineering polymeric compositions.

It would also be desirable to have a new class of polymeric compositions which could exhibit the thermosetting or thermoplastic properties and which could be polymerized with thermosetting polymers to impart processing advantages such as compression molding capabilities.

The present invention concerns a compound which comprises (a) a reactive arylcyclobutene moiety and (b) a molecular group bonded to the aryl group of the arylcyclobutene moiety.

According to the present invention, there are provided N-substituted arylcyclobutenyl-maleimides corresponding to the Formula I: 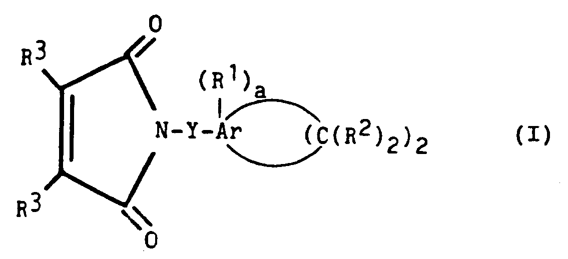
wherein
Ar is an aromatic radical;
R¹ is separately in each occurrence C₁₋₂₀alkyl, C₁₋₂₀ alkoxy, C₁₋₂₀ alkylthio, C₆₋₂₀ aryl, C₆₋₂₀ aryloxy, C₆₋₂₀ arylthio, C₇₋₂₀ alkaryl, C₇₋₂₀ alkaryloxy, C₇₋₂₀ alkarylthio, C₇₋₂₀ aralkyl, C₇₋₂₀ aralkoxy, C₇₋₂₀ aralkylthio, cyano, carboxylate, hydrocarbylcarbonyloxy, nitro, halo, hydrocarbylsulfinyl, hydrocarbylsulfonyl or amino;
R² is separately in each occurrence hydrogen, cyano, carboxylate, hydrocarbylcarbonyloxy, nitro, halo, hydrocarbylsulfonyl, hydrocarbylsulfinyl, alkyl, amido or hydrocarbyloxy;
R³ is separately in each occurrence hydrogen, C₁₋₂₀ alkyl, C₁₋₂₀ alkoxy, C₁₋₂₀ alkylthio, C₆₋₂₀ aryl, C₆₋₂₀ aryloxy, C₆₋₂₀ arylthio, C₇₋₂₀ alkaryl, C₇₋₂₀ alkaryloxy, C₇₋₂₀ alkarylthio, C₇₋₂₀ aralkyl, C₇₋₂₀ aralkoxy or C₇₋₂₀ aralkylthio;
Y is a divalent hydrocarbylene, hydrocarbylenecarbonyloxy, hydrocarbyleneoxy, hydrocarbyleneamino, hydrocarbylenecarbonyl, hydrocarbylenethio, hydrocarbylenepolythio, hydrocarbylenesulfinyl or hydrocarbylenesulfonyl; and
a is an integer of between 0 and 3.

Preferably, the N-substituted arylcyclobutenylmaleimides correspond to the Formula II: 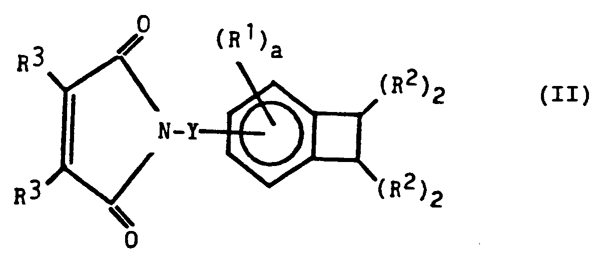
wherein
R¹, R², R³, Y and a are as defined above in connection with Formula I.

In the above formulae, R¹ is preferably C₁₋₂₀ alkyl, halo, nitro or cyano. Most preferably R¹ is C₁₋₃ alkyl, halo, nitro or cyano.

R² is preferably hydrogen, halo, nitro or cyano. R² is more preferably hydrogen or cyano and most preferably hydrogen.

R³ is preferably hydrogen, or C₁₋₂₀ alkyl. R³ is more preferably hydrogen or C₁₋₃ alkyl and most preferably hydrogen.

Y is preferably alkylene, arylene, alkylenebridged polyarylene, cycloalkylene-bridged polyarylene, alkylenecarbonyloxy, arylenecarbonyloxy, arylenecarbonyl, alkylenecarbonyl, aryleneoxy, alkyleneoxy, aryleneamino, alkyleneamino, alkylenethio, alkylenepolythio, arylenethio, arylenepolythio, arylenesulfinyl, alkylenesulfinyl, arylenesulfonyl, alkylenesulfonyl, alkylene-bridged polyarylenecarbonyloxy, cycloalkylene-bridged polyarylenecarbonyloxy, alkylene-bridged polyaryleneoxy, cycloalkylene-bridged poly-aryleneoxy, alkylenebridged polyarylenethio, cycloalkylene-bridged polyarylenethio, alkylene-bridged polyarylenesulfinyl, cycloalkylene-bridged polyarylenesulfinyl, alkylene-bridged polyarylenesulfonyl or cycloalkylene-bridged polyarylenesulfonyl. More preferred divalent organic radicals include alkylene, arylene, alkylenecarbonyloxy, arylenecarbonyloxy, alkyleneoxy, aryleneoxy, alkylenethio or arylenethio. Y is most preferably alkylene or arylene.

Ar is an aromatic radical. Aromatic as used herein refers to carbocyclic or heterocyclic rings in which 4n + 2 delocalized π electrons are contained in an orbital ring. This property is also known as resonance stabilization or delocalization.

Preferably, Ar is a benzene, naphthalene, phenanthrene, anthracene or biaryl radical, or two or more aromatic radicals bridged by alkylene or cycloalkylene moieties. Ar is more preferably benzene, naphthalene, biphenyl, binaphthyl, diphenylalkylene or diphenylcycloalkylene. Ar is most preferably a benzene radical.

Preferred heterocyclic aromatic radicals include pyrrole, furan, thiophene, imidazole, oxazole, thiaxole, pyrazole, pyridine, and pyrimidine radicals. More preferred heterocyclic aromatic radicals are pyridine, furan, and thiophene radicals, with pyridine radical being most preferred. The carbocyclic aromatic rings are preferred over the heterocyclic aromatic rings.

Hydrocarbyl means herein an organic radical containing carbon and hydrogen atoms. The term hydrocarbyl includes the following organic radicals: alkyl, alkenyl, alkynyl, cycloalkyl, cycloalkenyl, aryl, aliphatic and cycloaliphatic aralkyl and alkaryl. Aliphatic refers herein to straight- and branched-, and saturated and unsaturated, hydrocarbon chains, that is, alkyl, alkenyl or alkynyl. Cycloaliphatic refers herein to saturated and unsaturated cyclic hydrocarbons, that is, cycloalkenyl and cycloalkyl. The term aryl refers herein to biaryl, biphenylyl, phenyl, naphthyl, phenanthranyl, anthranyl and two aryl groups bridged by an alkylene group. Alkaryl refers herein to an alkyl-, alkenyl- or alkynyl-substituted aryl substituent wherein aryl is as defined hereinbefore. Aralkyl means herein an alkyl, alkenyl or alkynyl group substituted with an aryl group, wherein aryl is as defined hereinbefore. C₁₋₂₀ alkyl includes straightand branched-chain methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl and eicosyl groups. C₁₋₅ alkyl includes methyl, ethyl, propyl, butyl and pentyl.

Cycloalkyl refers to alkyl groups containing one, two, three or more cyclic rings. Cycloalkenyl refers to mono-, di- and polycyclic groups containing one or more double bonds. Cycloalkenyl also refers to cycloalkenyl groups wherein two or more double bonds are present.

Hydrocarbylene herein refers to a divalent hydrocarbon radical and is analogous to the hydrocarbyl radicals described hereinbefore with the single difference that the hydrocarbylene radical is divalent.

Hydrocarbyleneamido refers herein to a divalent radical wherein a hydrocarbylene radical is bonded to an amido group, and corresponds to the formula 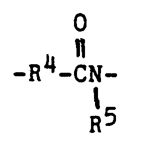
wherein R⁴ is a hydrocarbylene radical and R⁵ is hydrogen or a hydrocarbyl radical.

Hydrocarbyleneoxy refers herein to a divalent radical in which a hydrocarbylene radical is bonded to a divalent oxygen atom and corresponds to the formula-R⁴-O- wherein R⁴ is as defined hereinbefore.

Hydrocarbylenecarbonyloxy refers to a hydrocarbylene moiety which is bonded to a carbonyl moiety which is further bonded to a divalent oxygen atom and corresponds to the formula 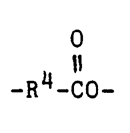
wherein R⁴ is as defined hereinbefore.

Hydrocarbylenethio refers herein to a radical in which a hydrocarbylene radical is further bonded to one or more sulfur moieties and corresponds to the formula -R⁴-(S)ₚ- wherein R⁴ is as hereinbefore defined, and wherein p is between 1 and 3.

Hydrocarbyleneamino refers herein to a hydrocarbylene radical bonded to an amino moiety and generally corresponds to the formula 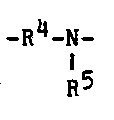
wherein R⁴ and R⁵ are as defined hereinbefore.

Hydrocarbylenesulfinyl refers herein to a hydrocarbylene moiety bonded to a sulfinyl moiety and generally corresonds to the formula 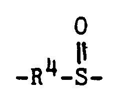
wherein R⁴ is as hereinbefore defined.

Hydrocarbylenesulfonyl generally corresponds to a radical in which a hydrocarbylene radical is bonded to a sulfonyl radical and corresponds to the formula 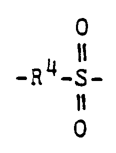
wherein R⁴ is as hereinbefore defined.

Wherein the bridging member is a hydrocarbyleneamido, hydrocarbyleneoxy, hydrocarbyleneamino, hydrocarbylenethio, hydrocarbylenecarbonyloxy moiety, the amido, amino, oxy, thio, sulfinyl or sulfonyl moiety is preferably bonded to the aryl portion of the arylcyclobutene.

Examples of preferred N-substituted benzocyclobutenyl maleimides include N-benzocyclobutenylmethyl maleimide, N-benzocyclobutenylethyl maleimide, N-benzocyclobutenylpropyl maleimide, N-benzocyclobutenylbutyl maleimide, N-benzocyclobutenylpentyl maleimide, N-benzocyclobutenylhexyl maleimide, N-benzocyclobutenylphenyl maleimide, N-benzocyclobutenylbiphenyl maleimide, N-benzocyclobutenyloxycarbonylmethyl maleimide, N-benzocyclobutenyloxycarbonylethyl maleimide, N-benzocyclobutenyloxycarbonylpropyl maleimide, N-benzocyclobutenyloxycarbonylbutyl maleimide, N-benzocyclobutenyloxycarbonylpentyl maleimide, N-benzocyclobutenyloxycarbonylhexyl maleimide, N-benzocyclobutenyloxycarbonylphenyl maleimide, N-benzocyclobutenyloxycarbonylbiphenyl maleimide, N-benzocyclobutenylthiomethyl maleimide, N-benzocyclobutenylthioethyl maleimide, N-benzocyclobutenylthiopropyl maleimide, N-benzocyclobutenylthiobutyl maleimide, N-benzocyclobutenylthiopentyl maleimide, N-benzocyclobutenylthiohexyl maleimide, N-benzocyclobutenylthiophenyl maleimide, N-benzocyclobutenylthiobiphenyl maleimide, N-benzocyclobutenyloxymethyl maleimide, N-benzocyclobutenyloxyethyl maleimide, N-benzocyclobutenyloxypropyl maleimide, N-benzocyclobutenyloxybutyl maleimide, N-benzocyclobutenyloxypentyl maleimide, N-benzocyclobutenyloxyhexyl maleimide, N-benzocyclobutenyloxyphenyl maleimide, N-benzocyclobutenyloxybiphenyl maleimide.

The arylcyclobutene moieties can be prepared by several synthesis schemes.

In one synthesis scheme, an alkyl-substituted aromatic compound which is further substituted with an aryl deactivating substituent is chloroalkylated in a position ortho to the alkyl group. In the preferred embodiment wherein the aromatic compound is benzene, the starting material corresponds to the following formula 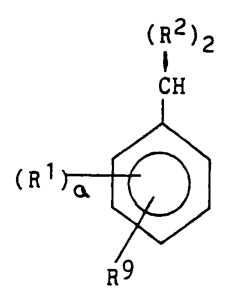
wherein R¹ and R² are defined hereinbefore; R⁹ is any aryl deactivating substituent; and a is an integer of 0, 1, 2, or 3. The alkyl N-substituted aromatic compound is chloroalkylated by contacting the alkyl aromatic compound with a chloroalkylating agent and thionyl chloride in the presence of an iron chloride catalyst so as to result in a product which contains a chloroalkyl group ortho to the alkyl substituent. In the embodiment wherein the aromatic compound is a benzene ring, the product corresponds to the formula 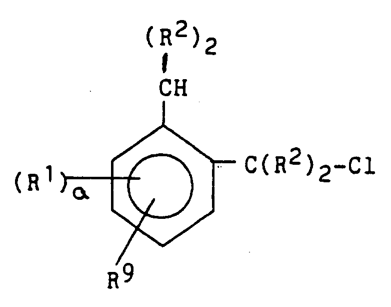
Preferably, R⁹ is a hydrocarbyloxycarbonyl, carboxamide, hydrocarbylcarbonyl, carboxylate, halocarbonyl, nitrile, nitro, sulfone or sulfoxide group. R⁹ is more preferably a halo or hydrocarbyloxycarbonyl group, with hydrocarbyloxycarbonyl being the most preferred group. Preferably a is 0 or 1, most preferably 0.

In this process the chloroalkylating agent is preferably chloromethyl methyl ether, although other chloroalkylating agents such as bis(chloromethyl) ether could be used. At least a 2:1 molar excess of the chloroalkylating agent to the alkyl-substituted aromatic compound is needed. It is preferable to use between a 6:1 and 3:1 ratio of chloroalkylating agent to alkyl aromatic compound. The catalyst is ferric chloride (FeCl₃) while the cocatalyst is thionyl chloride. The catalyst can be present in between 0.1 and 1.0 mole per mole of alkyl aromatic. More preferably between 0.2 and 0.4 mole of catalyst is present for each mole of alkyl aromatic compound. Preferably between 0.1 and 1.0 mole of thionyl chloride per mole of alkyl aromatic is used, more preferably between 0.2 and 0.4 mole per mole of alkyl aromatic.

This process can be performed at a temperature of between 40°C and 80°C, preferably 40°C and 60°C. Below 40°C, the reaction rate is low. The boiling point of some of the components of the reaction mixture starts at 80°C.

This process can be done by contacting the alkyl aromatic compound with the chloromethylating agent, catalyst and cocatalyst in a suitable solvent. Suitable solvents include chlorinated hydrocarbon solvents. Thereafter the reaction mixture is heated to the appropriate temperature.

The product can be recovered by quenching the reaction mixture with alcohols or water to inactivate the chloroalkylating agents remaining, stripping off the volatiles and washing cut the catalyst with water. The product thereafter is recovered by distillation.

The ortho chloroalkylated alkyl aromatic compounds can be converted to aromatic compounds with cyclobutene rings fused thereto, by pyrolysis. This is achieved by contacting the ortho chloroalkylated alkyl aromatic compound with at least 2 times its weight of a suitable diluent, and thereafter passing the mixture through a reactor at a temperature of 550°C or greater and a pressure of between atmospheric and 25 mm of mercury (3 KPa). Suitable diluents are generally substituted aromatic compounds which are inert to the chloromethylated alkyl aromatic compound and are stable at pyrolysis temperatures. Examples of suitable diluents are benzene, toluene, xylenes, chlorobenzenes, nitrobenzenes, methylbenzoates, phenyl acetate or diphenyl acetate. Preferred diluents are the xylenes. Preferable temperatures are between 700°C and 750°C. Preferable pressures are between 15 and 25 mm of mercury (2-3 KPa). In a preferred embodiment, the reaction mixture is passed through a hot tube packed with an inert material, for example, quartz chips or stainless steel helices. The product can be recovered by distillation. The product wherein the aromatic compound is benzene corresponds to the formula 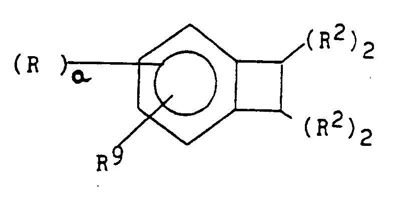
wherein R¹, R², R⁹ and a are as hereinbefore defined.

In the preferred embodiment wherein R⁹ is a hydrocarbyloxy carbonyl moiety, the hydrocarbyloxy carbonyl moiety can be converted to a carboxylate moiety by contacting the substituted (arylcyclobutene) compound with at least a molar equivalent of alkali metal hydroxide in an alkanol-water solvent system. In the embodiment wherein the aromatic radical is benzene, the product corresponds to the formula 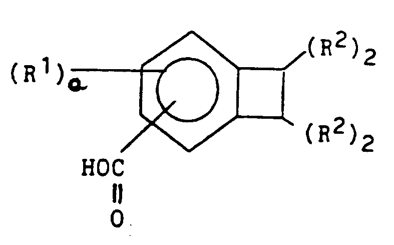
Thereafter the carboxylate-substituted (arylcyclobutene) compound can be converted to an acid chloride by contacting the carboxylate-substituted (arylcyclobutene) compound with thionyl chloride and refluxing at 70°C to 80°C. The acid halide-substituted (arylcyclobutene) so formed can be used to prepare the novel monomers of this invention, as described hereinafter. In the embodiment wherein the aryl radical is a benzene ring, the product corresponds to the formula 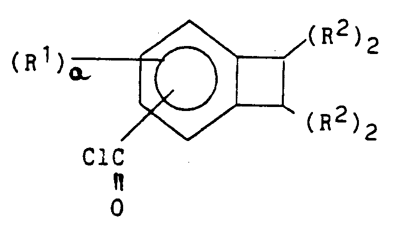
In an alternative synthesis, an aryl compound with ortho dibromomethyl groups can be converted to a 1,2-diiodoarylcyclobutene, by contacting the aryl compound substituted with ortho dibromomethyl moieties with an alkali metal iodide in an alkanol solvent at reflux so as to form the diiodoarylcyclobutenes. The product can be recovered by filtering, evaporating the filtrate and recrystallising the product. In the embodiment wherein the aryl radical is a benzene radical, the starting material corresponds to the formula 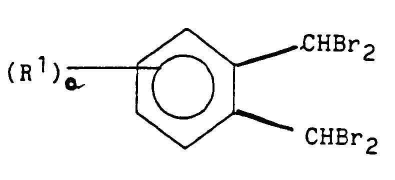
and the iodobenzocyclobutene corresponds to the formula 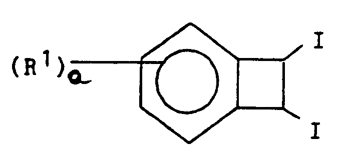
The 1,2-diiodoarylcyclobutenes can be converted to arylcyclobutenes by dissolving the 1,2-diiodoarylcyclobutenes in an alcohol solvent, preferably methanol or ethanol, and contacting the solution with an alkali metal hydroxide in the presence of a palladium-on-carbon catalyst and H₂ gas at a temperature of 20°C to 30°C. In general, at least between 2 and 4 moles of alkali metal hydroxide per mole of 1,2-diiodoarylcyclobutene is used. Preferably, between 50 and 200 psi (350-1400 KPa) of hydrogen gas is used. The arylcyclobutenes prepared in this manner can be recovered by distillation. In the embodiment wherein the aryl radical is a benzene radical, the product corresponds to the formula 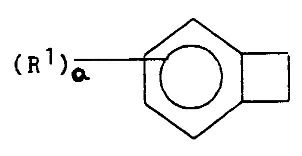
The arylcyclobutene is thereafter brominated. In this process, the arylcyclobutene is dissolved in acetic acid and contacted with a brominating agent of pyridinium hydrobromide perbromide in the presence of mercuric salts, for example, mercuric acetate, at a temperature of between 20°C and 50°C. The brominated product can be recovered by extraction and distillation. In the embodiment wherein aryl radical is benzene, the product corresponds to the formula 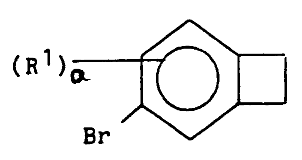
The brominated arylcyclobutene can thereafter be carbonylated to prepare a hydrocarbyloxy carbonylsubstituted arylcyclobutene. This carbonylation is achieved by dissolving the brominated arylcyclobutene in an alkanol solvent, and thereafter contacting the solution with carbon monoxide under pressure in the presence of a palladium catalyst, wherein the palladium is in the zero valence state, in the further presence of an acid acceptor under conditions such that the brominated arylcyclobutene compound undergoes carbonylation. Preferred catalysts are palladium acetate with a cocatalyst of triphenyl phosphine, palladium triphenyl phosphine tetrakis, andbis(triphenyl phosphine) palladium chloride complex. The acid acceptor is generally a tertiary amine. In general, the reaction vessel is pressurized with carbon monoxide to a pressure of between atmospheric and 3000 psi (20 MPa), preferred pressures are between 600 and 1000 psi (4-7 MPa).

This process is preferably run at a temperature of between 100°C and 140°C, most preferably between 120°C and 130°C. The hydrocarbyloxycarbonyl arylcyclobutene can be recovered by filtering off the catalyst, washing away the acid scavenger with a 10 percent strong acid solution, stripping off the solvent and distilling the product to purify it. To prepare a carboxamide-substituted arylcyclobutene, a primary or secondary amine is substituted for the alcohol solvent. In the embodiment wherein the aryl radical is a benzene radical, the process corresponds to the following equation: 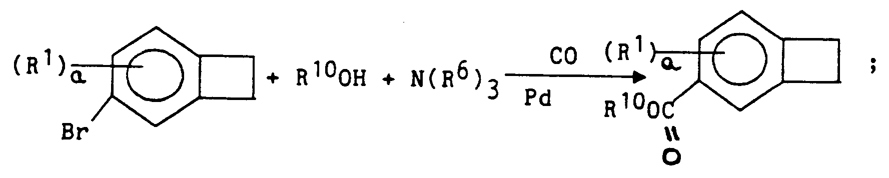
wherein R¹ and a are as hereinbefore defined and R⁶ and R¹⁰ are hydrocarbyl moieties. The hydrocarbyloxycarbonyl-substituted or carboxamide-substituted arylcyclobutenes can thereafter be acidified and converted to acid chlorides by the process described hereinbefore.

In another preparation of an arylcyclobutene, the reaction may follow that reported by Skorcz and Kaminski, Org. Syn., 48, pages 53-56 (1968). In a typical preparation, an alkyl cyanoacetate is added to a solution of sodium metal in ethanol followed by the addition of an ortho-halomethylaryl halide. The alkyl 2-(o-haloarylmethyl)cyanoacetate is isolated and treated with aqueous sodium hydroxide. Subsequent acidification results in the cyanoacetic acid derivative. That derivative is placed into N,N-dimethylformamide and is refluxed to form the 3-(o-haloaryl)propionitrile derivative which is isolated and added to a suspension of sodamide in liquid ammonia. After an appropriate reaction time, ammonium nitrate is added and the ammonia allowed to evaporate. The cyanoarylcyclobutene is isolated by ether extraction and purified by fractional distillation under reduced pressure.

Substituted arylcyclobutenes can be prepared by the same technique by using the appropriately substituted reactants, such as an alkyl or alkoxybenzyl halide. Also substituents can result from using an alkyl haloacetate or a dialkylmalonate.

In another preparation based on the paper by Matsura et al., Bull. Chem. Soc. Jap., 39, 1342 (1966), o-aminoaryl carboxylic acid is dissolved in ethanol and hydrochloric acid added. Isoamylnitrite is slowly added to the cold stirred solution and diethyl ether is then added. The product, aryldiazonium-2-carboxylate hydrochloride, is filtered. That product is placed in a solvent, preferably ethylene dichloride, and acrylonitrile and propylene oxide is added to the stirred mixture which is then heated under nitrogen until the reaction is complete. After cooling, the mixture is filtered and the product, 1-cyanoarylcyclobutene, is isolated by fractionally distilling the filtrate under reduced pressure.

Amounts of reactants, reaction parameters and other details can be found in the cited article, the examples of this application, or can be easily deduced therefrom.

In a next sequence of reactions, the cyanoarylcyclobutene or substituted derivative is nuclear substituted. In one preparation, the cyanoarylcyclobutene is added slowly to a cold solution of sodium nitrate in concentrated sulfuric acid to form 5-nitro-1-cyanoarylcyolobutene. That nitro compound is isolated, dissolved in ethanol and reduced by hydrogenation over a palladium on carbon catalyst. The isolated product is 5-amino-1-cyanoarylcyclobutene. In the preferred embodiment where the aryl radical is benzene, the product corresponds to the formula 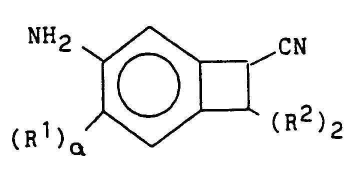
Cyclobutapyridines are prepared by the pyrolysis of 4-pyridyl propargyl ether at 550°C. See J. M. Riemann et al, Tetrahedron Letters, No. 22, pp. 1867- 1870 (1977). Alternatively, a pyridine-4-carbonitrile with an alkyl substituent on the carbon atom adjacent to the nitrile is reacted with sodium azide and ammonium chloride in N,N-dimethylformamide to prepare a 5-(alkyl-4-pyridyl)tetrazole. The 5-(alkyl-4-pyridyl)tetrazole is pyrolyzed at about 600°C to prepare a cyclobutapyridine. See. W. D. Crow et al. Australian Journal of Chemistry 1741 et seq. (1975).

Amino cyclobutapyridines are prepared by reacting a cyclobutapyridine with sodamide (NaNH₂) in N,N-dimethylaniline solvent at 110°C. A hydroxycyclobutapyridine is prepared by reacting one mole of an aminocyclobutapyridine with one mole of sodium nitrite and two moles of sulfuric acid in water at 0°C for a period of time and thereafter warming to 50°C. Halo-substituted cyclobutapyridine is prepared by reacting a hydroxypyridine in thionyl at reflux either neat or in solution, for example, thionyl chloride or thionyl bromide, in N,N-dimethylformamide solvent.

To prepare an N-substituted arylcyclobutenyl cyclic imide with a hydrocarbylene amido, hydrocarbyleneoxy or hydrocarbyleneoxycarbonyl bridge, a maleic anhydride is reacted with a hydrocarbon substituted with amino and carboxyl moieties, for the hydrocarbylene amido-bridged species, or a hydrocarbon substituted with amino and hydroxyl moieties, for the hydrocarbyleneoxy and hydrocarbyleneoxycarbonyl-bridged species, to prepare an amido alkanoic acid wherein the amido nitrogen is substituted with a carboxy-substituted hydrocarbyl or hydroxy-substituted hydrocarbyl moiety. This reaction can be performed at a temperature of between -40°C and 100°C in a suitable solvent. Suitable solvents include aliphatic hydrocarbons, aromatic hydrocarbons, ethers and halogenated hydrocarbons. It is preferred to run the process under an inert atmosphere. It is preferred to use freshly sublimed anhydride as any impurities can result in very poor yields. It is preferred to use at least a 5 percent excess of anhydride so as to drive the reaction to completion.

In the embodiment wherein the anhydride is maleic anhydride, these reactions are exemplified by the following equations 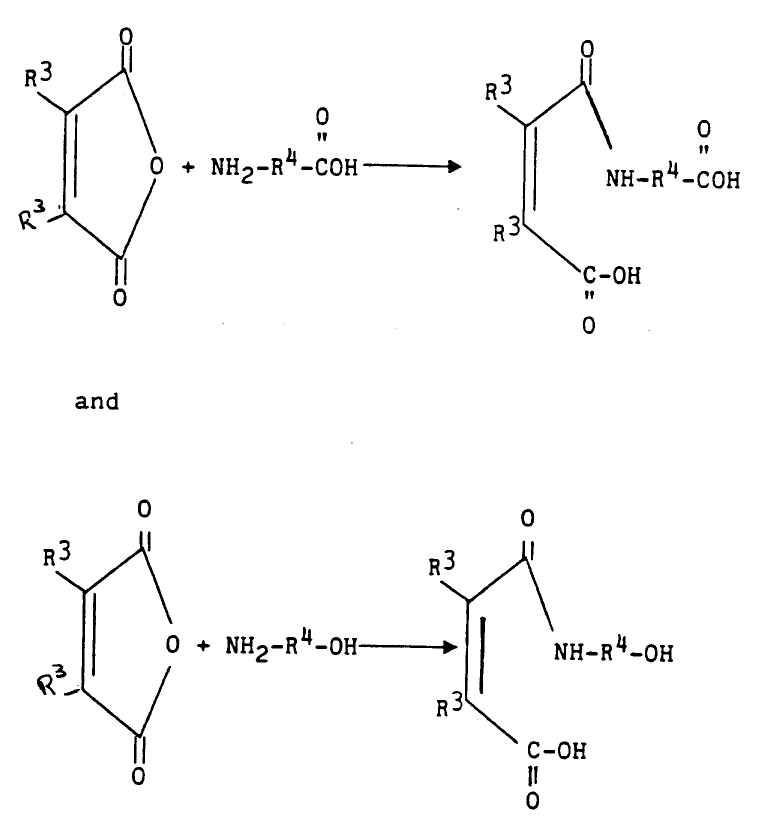
wherein R³ is as hereinbefore defined and R⁴ is a hydrocarbylene radical.

The amido alkenoic acid can be dehydrated using one of the two dehydration methods described hereinafter so as to prepare a N-substituted cyclic imide wherein the substituent is a N-hydrocarbylcarbonyloxycarbonyl maleimide, or a N-hydrocarbylcarbonyloxy meleimide.

In the preferred embodiment, the amido alkenoic acid is contacted with a dehydrating agent in an aprotic reaction medium in the presence of a nickel II salt catalyst. In general, the reaction medium is an aprotic solvent and can include ketones, ethers, amides or aliphatic halogenated hydrocarbons. Preferred reaction media include the ketones, with acetone being most preferred. The dehydrating agents include anhydrides, carbodiimides and isocyanates; with the anhydrides being preferred and acetic anhydride being most preferred.

The catalyst is any nickel II salt with nickel II acetate being most preferred. In general, between 1 and 5 percent of the catalyst is useful. It is preferable to run this process in the presence of an aprotic base such as a carbonate or tertiary amine, preferably a tertiary amine. In general, between 20 and 200 mole percent of a tertiary amine is used, with between 100 and 150 mole percent being preferred, wherein mole percentages are based on the starting amido alkenoic acid. The mole ratio of the dehydrating agent to the amido alkanoic acid is between 4:1 and 1:1, preferably between 1.5:1 and 1:1.

It is preferred to run this process under an inert atmosphere. Temperatures which are useful are those at which the dehydration takes place. Preferable temperatures are between -20°C and 100°C, with between 15°C and 25°C being most preferred.

In this reaction the amido alkenoic acid is not soluble in the reaction-medium but the cyclic imide product is soluble. The reactant is slurried in the reaction media and exposed to the reaction conditions described. The completion of the reaction is noted by dissolution of the reactants indicating formation of products.

In an alternative procedure, the amido alkenoic acid can be dehydrated by dispersing the compound in a glacial acetic acid reaction media in the presence of an alkali or alkaline earth metal acetate salt, and heating the reaction mixture to a temperature at which the dehydration takes place to form the cyclic imide rings. Generally, a sufficient amount of alkali or alkaline earth metal acetate salt to cause complete dehydration is suitable. Preferably, at least an equimolar amount of alkali or alkaline earth metal acetate salt is used, most preferably an excess of 5 mole percent. The process can be run at any temperature at which the dehydration takes place, preferable temperatures are between 50°C and 140°C, with between 100°C and 120°C being most preferred. Completion of the reaction is indicated by dissolution of the product.

In both instances, the product can be recovered by washing with water and thereafter an aqueous solution of an inorganic base.

The reaction is exemplified by the following equations 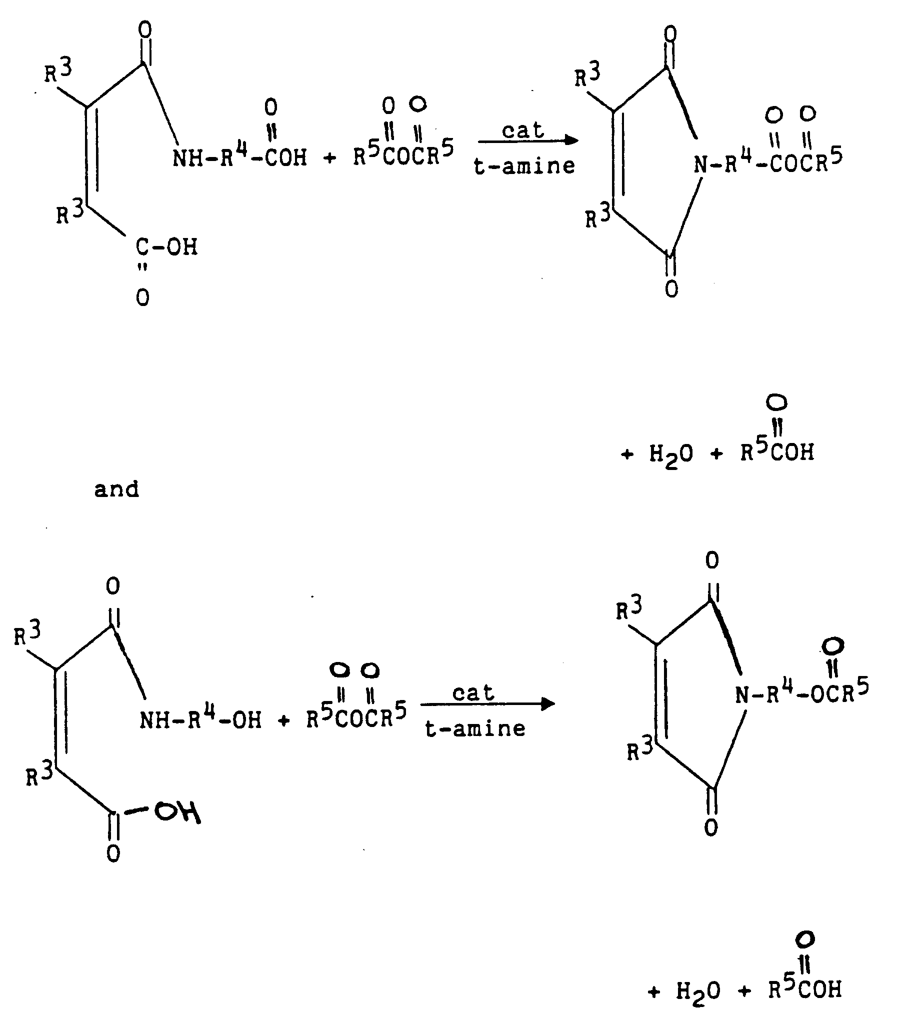
wherein R³ and R⁴ are as hereinbefore defined and R⁵ is a hydrocarbyl moiety.

The N-hydrocarbylcarbonyloxycarbonyl maleimide is converted to a hydrocarbylene amido-bridged N-substituted arylcyclobutenyl maleimide by reacting the N-hydrocarbylcarbonyloxycarbonyl maleimide with an amino-substituted arylcyclobutene in the presence of a tertiary amine. This process can be accomplished by contacting the starting reactants in a chlorinated aliphatic hydrocarbon solvent at about 0°C with agitation under an inert atmosphere. This process is exemplified by the following equation 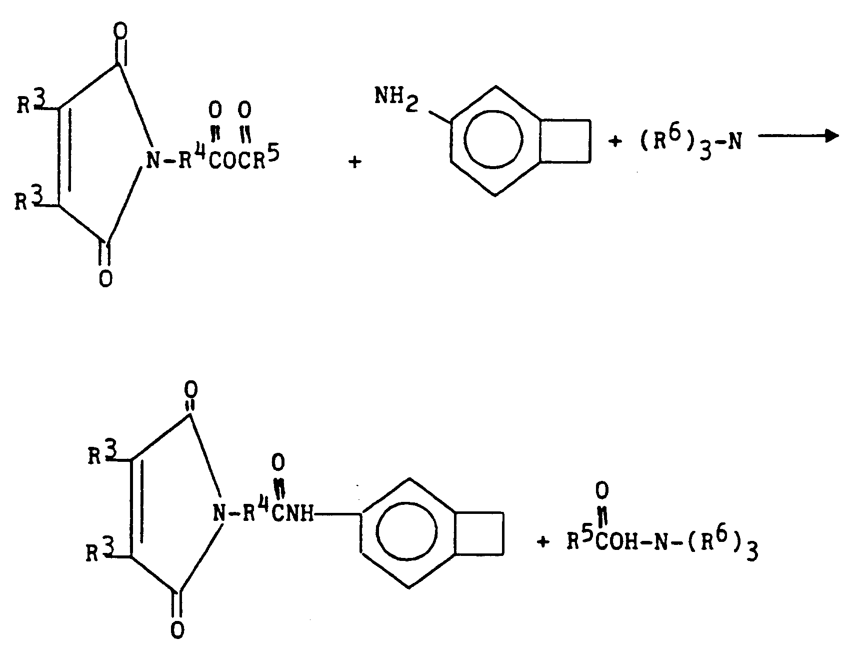
wherein R⁴ and R⁵ are as hereinbefore defined, and R⁶ is a hydrocarbyl radical.

To prepare a hydrocarbyleneoxy or hydrocarbyleneoxycarbonylbridged N-substituted arylcyclobutenyl maleimide, the N-hydrocarbylcarbonyloxyhydrocarbyl maleimide is hydrolyzed to prepare a N-hydroxyhydrocarbyl cyclic imide. The hydrolysis is usually run in an aqueous/alkanol solvent system in the presence of an acid or base catalyst at between room temperature and reflux of the solvent mixture (20°C to 60°C). This reaction is exemplified by the following equation 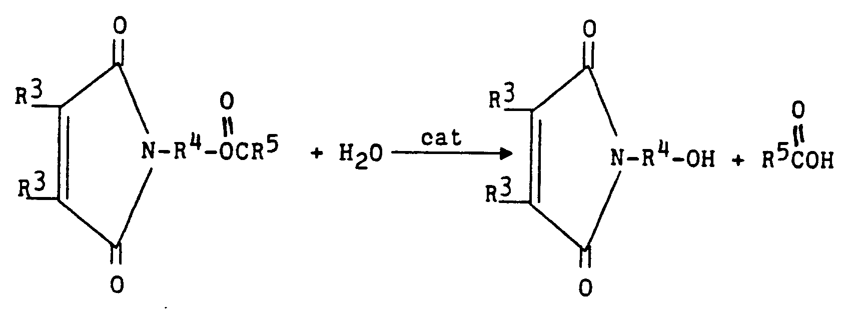
wherein R³, R⁴ and R⁵ are as hereinbefore defined.

To prepare the hydrocarbyleneoxycarbonylbridged N-substituted arylcyclobutenyl maleimides, the N-hydroxyhydrocarbyl maleimide is reacted with a chlorocarbonyl-substituted arylcyclobutene. In practice, the N-hydroxyhydrocarbyl maleimide is dissolved in a chlorinated aliphatic hydrocarbon solvent to which is added a tertiary amine, which functions as an acid acceptor, and thereafter the chlorocarbonyl-substituted arylcyclobutene in a chlorinated aliphatic hydrocarbon is added slowly to the mixture. This is preferably done at 0°C in an inert atmosphere. It is preferred to stir the reaction mixture for a period of time at 0°C after the addition is complete. This reaction is exemplified by the following equation 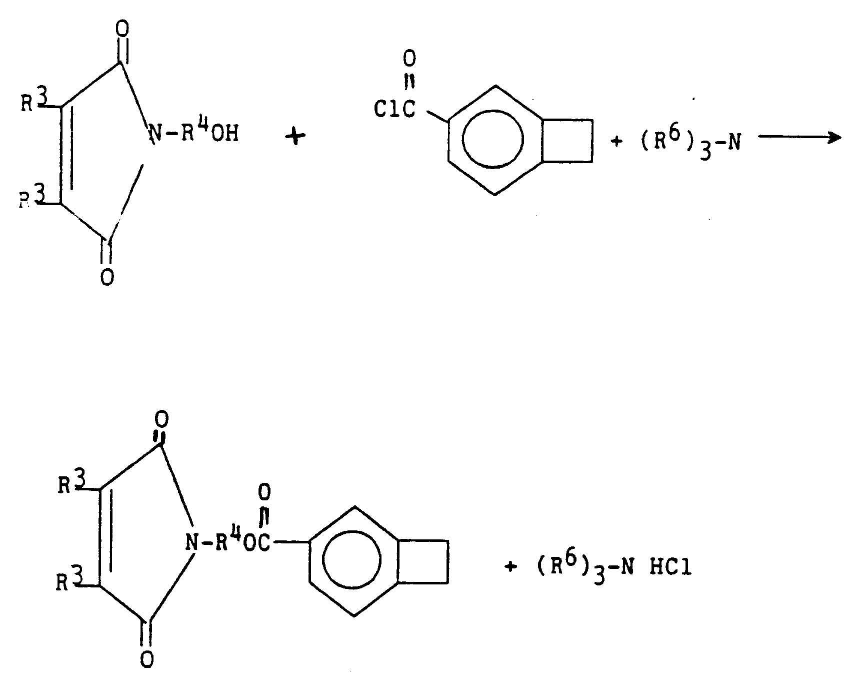
wherein R³, R⁴ and R⁶ are as hereinbefore defined.

The hydrocarbyleneoxy-bridged N-substituted arylcyclobutenyl maleimides can be prepared from the N-hydroxyhydrocarbyl maleimide in the following manner. The N-hydroxyhydrocarbyl maleimide is reacted with p-toluene sulfonyl chloride and pyridine to prepare a maleimide hydrocarbyl p-toluene sulfonate. Either excess pyridine or methylene chloride are used as the solvent. The reactants are contacted in equimolar amounts, unless pyridine is the solvent, at a temperature of between 0°C and 25°C. This reaction is exemplified by the following equation 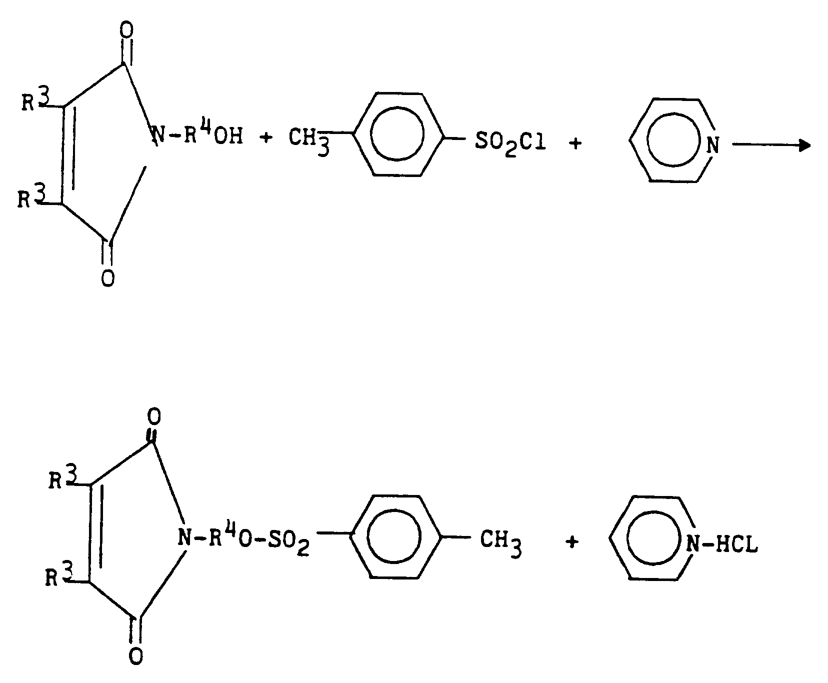
wherein R³ and R⁴ are as hereinbefore defined.

The maleimide hydrocarbyl p-toluene sulfonate is contacted with a hydroxy-substituted arylcyclobutene in the presence of a four to five molar excess of an alkali metal carbonate (such as potassium carbonate) based on the sulfonate, in a N,N-dimethyl formamide solvent, to prepare a hydrocarbyloxy-bridged N-substituted arylcyclobutenyl maleimide. This reaction takes place at temperatures of between 20°C and 140°C. This process is exemplified by the following equation 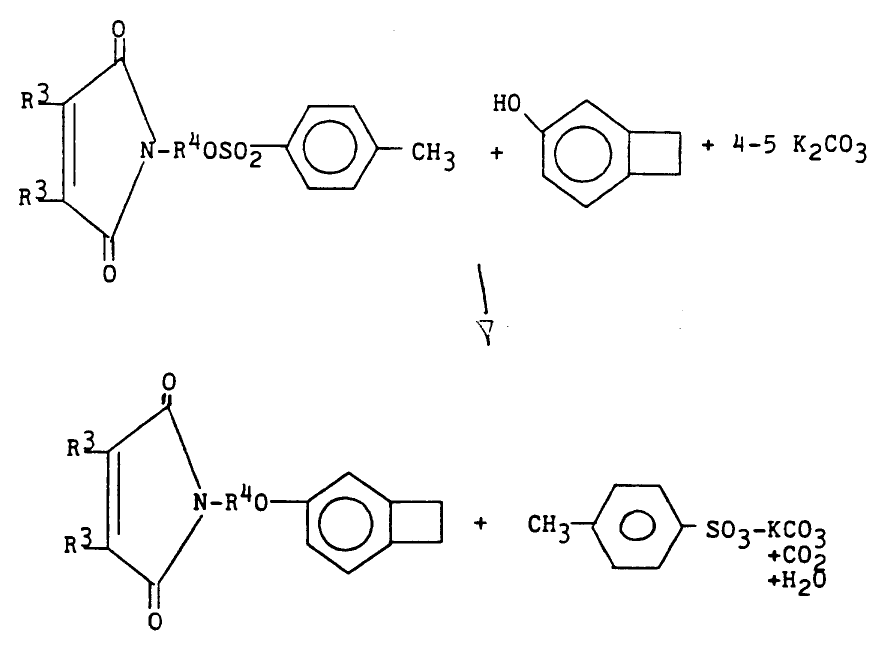
wherein R³ and R⁴ are as hereinbefore defined.

The hydrocarbylene amino-bridged N-substituted arylcyclobutenyl maleimides can be prepared by the following procedure. An amino-substituted arylcyclobutene is reacted with about an equimolar amount of a hydrocarbon substituted with aldehyde and nitro moieties, in the presence of between 0.3 to 1.5 moles of sodium cyanoborohydride in a methanolic solvent at 20°C to 25°C. The product is nitrohydrocarbyl amino-substituted arylcyclobutene. The process can be exemplified by the following equation 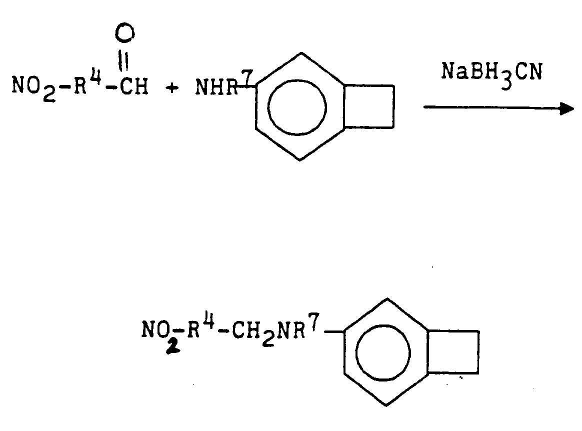
wherein R⁴ is as hereinbefore defined and R⁷ is hydrogen or a hydrocarbyl moiety. The nitro moiety on the nitrohydrocarbyl amino-substituted arylcyclobutene is reduced to an amine moiety by contacting with an excess of metallic zinc in a concentrated hydrochloric acid solution at between 20°C and reflux. The product corresponds to the formula 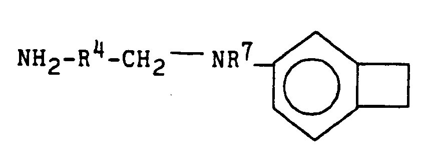
wherein R⁴ is as hereinbefore defined. The aminohydrocarbyl amino-substituted arylcyclobutene is thereafter reacted with a maleic anhydride to prepare a hydrocarbylene amino-bridged N-arylcyclobutenyl amido alkenoic acid. The cyclic anhydride and aminohydrocarbyl amino-substituted arylcyclobutene are contacted in a suitable solvent at a temperature of between -40°C and 100°C. Suitable solvents include aliphatic hydrocarbons, aromatic hydrocarbons, ethers and halogenated hydrocarbons. It is preferred to run the process under an inert atmosphere. It is also preferred to use freshly sublimed anhydride as any impurities in the anhydride can result in very poor yields. It is also preferred to use at least a 5 percent excess of anhydride so as to drive the reaction to completion with respect to the aminohydrocarbyl amino-substituted arylcyclobutene compound.

Preferred temperatures are between 0°C and 50°C with between 20°C and 25°C being most preferred.

This reaction is exemplified by the following equation 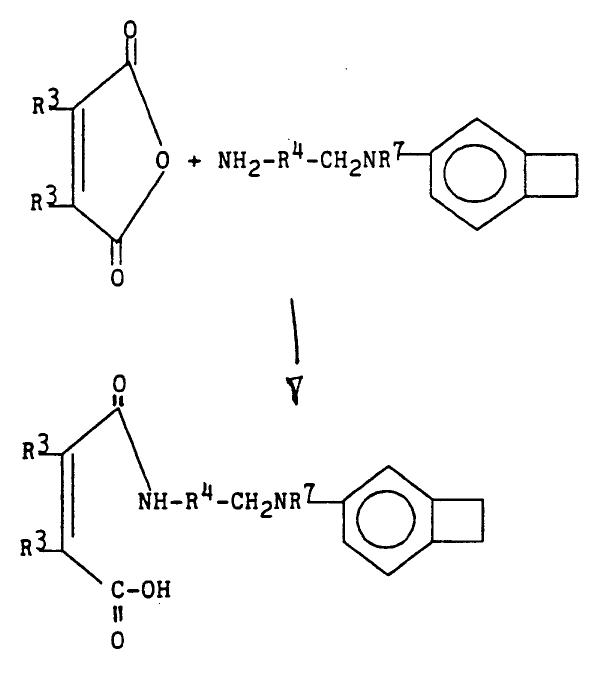
The hydrocarbylene amino-bridged N-aryl cyclobutenyl amido alkenoic acid is thereafter dehydrated by one of the methods described hereinbefore to prepare the hydrocarbylene amino-bridged N-substituted arylcyclobutenyl maleimide. This product corresponds to the formula 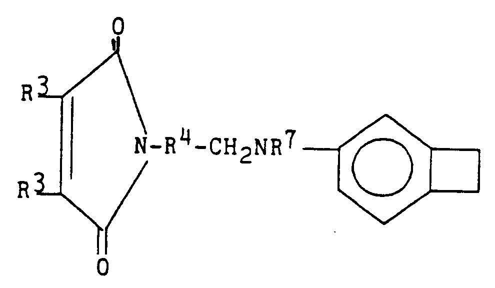
wherein R⁴ and R⁷ are as hereinbefore defined.

A hydrocarbylene-bridged N-substituted arylcyclobutenyl maleimide can be prepared by the follow ing procedure. A carboxy-substituted or carboxyhydrocarbyl-substituted arylcyclobutene is reduced to a hydroxyhydrocarbyl-substituted arylcyclobutene by reacting the starting material with a 3:1 molar excess of diborane in an ether or cyclic ether solvent at between 0°C to 20°C. This process is exemplified by the following equation 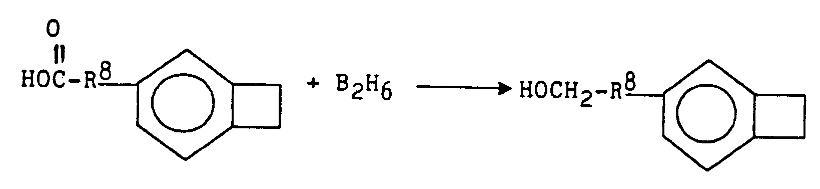
wherein R⁸ is a direct bond or a hydrocarbylene moiety. The hydroxyhydrocarbyl-substituted arylcyclobutene is reacted with a slight excess of thionyl chloride to prepare a chlorohydrocarbyl-substituted arylcyclobutene. The reactants are usually contacted neat or in a methylene chloride solvent at a temperature of between 0°C and 50°C. An example of the product corresponds to the formula 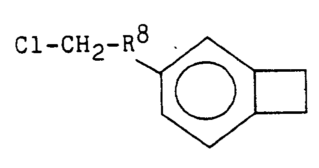
The chlorohydrocarbyl-substituted arylcyclobutene is thereafter reacted with about an equimolar amount of potassium phthalamide to prepare an N-arylcyclobutenylhydrocarbyl phthalamide. The reactants are generally contacted neat at temperatures of between 100°C and 200°C. This reaction is exemplified by the following equation 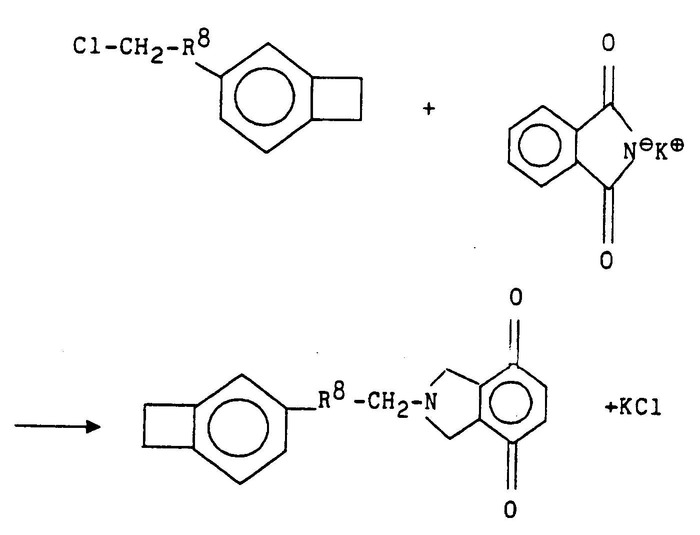
wherein R⁸ is as hereinbefore defined. The N-arylcyclobutenylhydrocarbyl phthalamide is reacted with about one equivalent of hydrazine hydrate to prepare an aminohydrocarbyl-substituted benzocyclobutene. The reactants are contacted in an alkanol solvent at the reflux of the solvent. The product corresponds to the formula 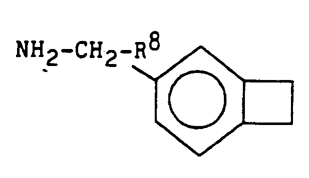
wherein R⁸ is as hereinbefore defined. The aminohydrocarbyl-substituted benzocyclobutene is thereafter reacted with a maleic anhydride to prepare an N-hydrocarbylarylcyclobutenyl amido alkenoic acid under the conditions described hereinbefore. This process is exemplified by the following equation 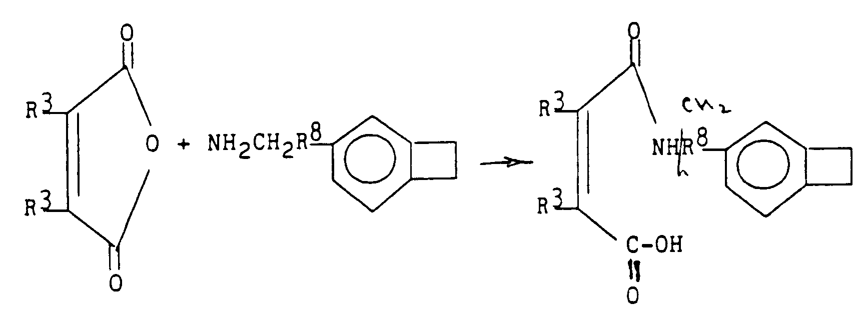
wherein R³ and R⁸ are as hereinbefore defined. The N-hydrocarbylarylcyclobutenyl amido alkenoic acid is then dehydrated to form a maleimide ring thus preparing an N-hydrocarbylarylcyclobutenyl maleimide. This process is performed using one of the two dehydration processes described hereinbefore.

To prepare a mercaptoarylcyclobutene, an arylcyclobutene sulfonic acid and equimolar amounts of sodium hydroxide are contacted in aqueous solution at 20°C-25°C to prepare sodium arylcyclobutene sulfonate. The sodium arylcyclobutene sulfonate is dried at 100°C, and thereafter contacted in neat form with about 0.48 mole of phosphorous pentachloride at 170°C to 180°C to prepare an arylcyclobutene sulfonyl chloride. The arylcyclobutene sulfonyl chloride is reduced with zinc, about 4.9 moles, in the presence of 6.8 moles of concentrated sulfuric acid at 0°C to prepare the mercaptoarylcyclobutene.

To prepare the alkylenethio-bridged N-substituted arylcyclobutenyl-maleimide, equimolar amounts of a mercapto arylcyclobutene, sodium hydroxide and a dihaloalkane are contacted in an alkanol solvent at between 0°C and 50°C. The product is a haloalkyl-substituted arylcyclobutenyl sulfide. This reaction is exemplified by the following equation 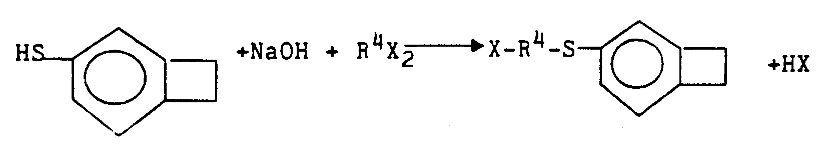
wherein X is halogen and R⁴ is a divalent alkane radical. Two moles of the haloalkyl-substituted arylcyclobutenyl sulfide is contacted with 0.8 moles of potassium phthalimide and 0.4 mole of potassium carbonate. The reactants are contacted neat at a temperature of 190°C to prepare an n-phthalimidoalkyl arylcyclobutenyl sulfide. This process is exemplified in one preferred embodiment by the following equation 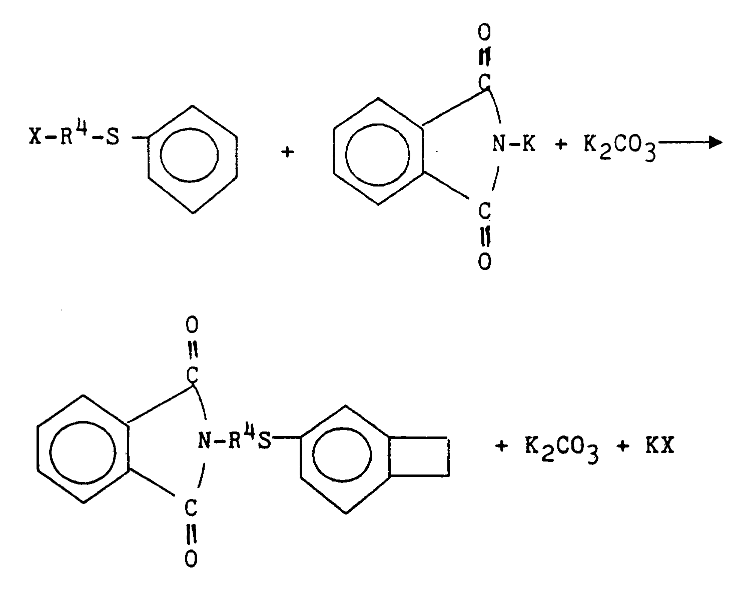
The phthalimidoalkyl arylcyclobutenyl sulfide is contacted with a hydrazine hydrate in a mole ratio of 1 to 1.25, respectively, in an alkanol solvent at reflux to prepare an aminoalkyl arylcyclobutenyl sulfide. In one preferred embodiment, the product corresponds to the formula 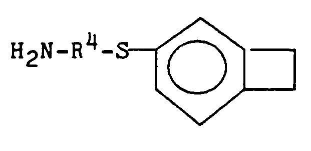
The aminoalkylarylcyclobutenyl sulfide is then reacted with an unsaturated cyclic anhydride to prepare a thioalkylene-bridged N-aryl cyclobutenyl aminoalkenoic acid. This is achieved under conditions described hereinbefore. Thereafter, the alkylenethio N-arylcyclobutenyl amidoalkenoic acid can be dehydrated by one of the methods described hereinbefore to prepare an alkylenethio-bridged N-substituted arylcyclobutenyl cyclic imide.

To prepare arylenethio-bridged N-arylcyclobutenyl maleimide, equimolar amounts of a mercapto arylcyclobutene, sodium hydroxide and a halonitro-substituted aromatic compound are contacted in an alkanol solvent under reflux to prepare a nitroaryl arylcyclobutenyl sulfide. The nitro group on the nitroaryl arylcyclobutenyl sulfide is reduced by contacting one mole of such compound with about two moles of tin and about six moles of concentrated hydrochloric acid to prepare an aminoaryl arylcyclobutenyl sulfide. The aminoaryl arylcyclobutenyl sulfide is thereafter contacted with an unsaturated cyclic anhydride in equimolar amounts in methylene chloride at a temperature of 0°C to 25°C to prepare an arylenethiobridged N-arylcyclobutenyl amidoalkenoic acid. The arylenethio-bridged N-arylcyclobutenyl amidoalkenoic acid is dehydrated using procedures described hereinbefore to prepare an arylenethio-bridged N-arylcyclobutenyl maleimide.

The hydrocarbylenethio-bridged N-arylcyclobutenyl maleimides can be contacted with equimolar amounts of peracetic acid in an ethyl acetate solvent at between 0°C to 20°C to prepare a hydrocarbylenesulfinyl-bridged N-arylcyclobutenyl maleimide. The hydrocarbylenethio-bridged N-arylcyclobutenyl maleimide can be contacted with about 2 moles of peracetic acid for each mole of the bridged cyclic imide in ethyl acetate solvent at about 0°C to 20°C to prepare a hydrocarbylenesulfonyl-bridged N-arylcyclobutenyl maleimide.

To prepare the various bridged N-arylcyclobutenyl maleimides wherein the aryl moiety is a heterocycle, the appropriately substituted heterocyclic N-arylcyclobutenyl maleimide is reacted in the manner described herein to get the appropriately desired compound.

The compounds of this invention are unique in several respects. They have intramolecular diene and dienophile functionality. They are thermally stable for long periods at elevated temperatures, up to 100°C. They are readily polymerizable. The compounds of this invention are useful in the preparation of polyimides by polymerization of one or more of the compounds of this invention. It is believed that the polymerization takes place by a Diels-Alder reaction wherein the unsaturation on the cyclic imide acts as a dienophile while the cyclobutene ring forms a diene which reacts with the dienophile to form the polymeric compositions.

The polymers of this invention are prepared by heating the compounds described hereinbefore to a temperature of 170°C or greater. Preferable temperatures for polymerization are 200°C or greater. In general, it is preferable to run the polymerization at a temperature of between 170°C and 300°C, with between 200°C and 300°C being most preferred.

It is believed that the polymeric composition contains units which correspond to the formula 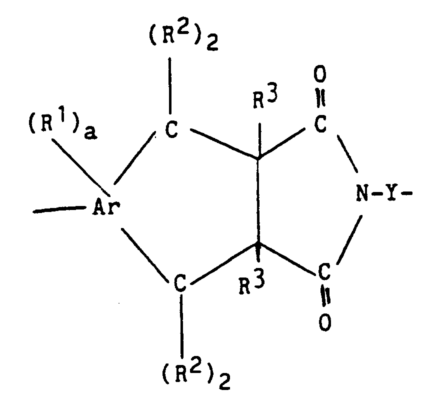
It is further believed that in one preferred embodiment the polymers derived from monomers of such a formula result in the preparation of polymers which correspond generally to the formula 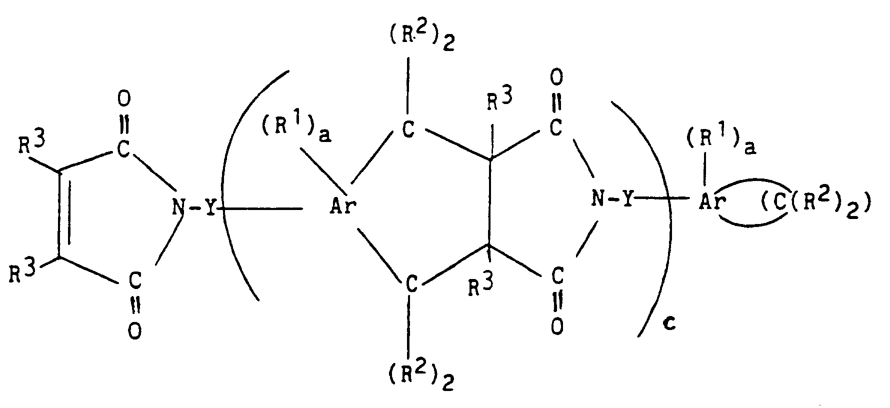
wherein Ar, R¹, R², R³, Y and a are as described hereinbefore and c is a real number of 2 or greater, and most preferably 20 or greater.

In another preferred embodiment, the polymeric composition is the polymer of one or more compounds which corresponds to the formula 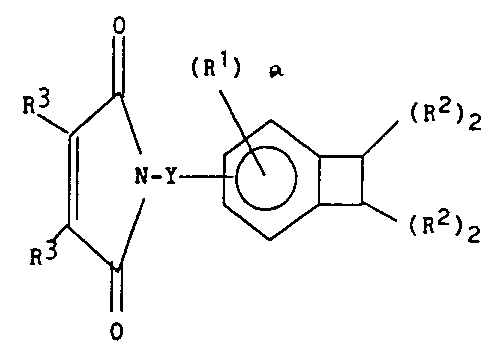
wherein R¹, R², R³, Y and a are as hereinbefore defined. In this embodiment, it is believed that the polymer prepared contains units which correspond to the formula 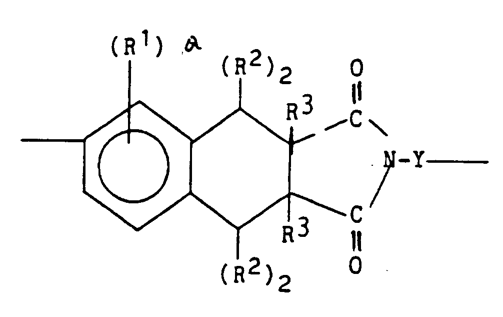
wherein R¹, R², R³, Y and a are as hereinbefore defined.

In one preferred embodiment wherein the compound or compounds polymerized corresponds to said formula, it is believed that the polymer prepared corresponds to the formula 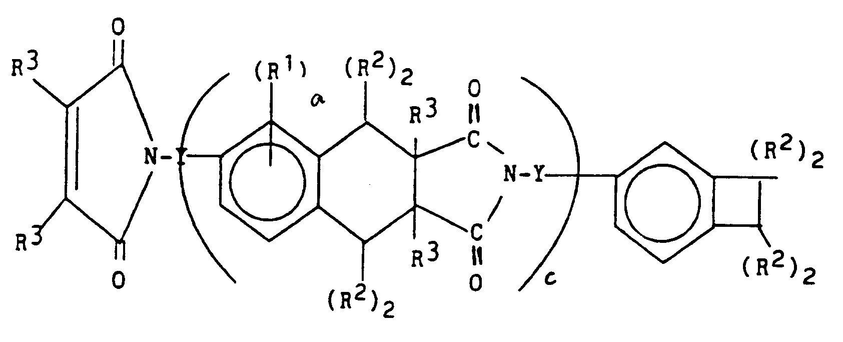
wherein R¹, R², R³, Y and a are as hereinbefore defined, and c is a real number of 2 or greater. c is preferably 20 or greater.

The novel N-substituted arylcyclobutenylmaleimide compounds of this invention are useful in the preparation of polymeric compositions. In general, these polymeric compositions are prepared by contacting these N-substituted arylcyclobutenyl maleimide compounds and heating them to the polymerization temperature of the particular monomer used. The polymerization is an addition polymerization wherein no volatiles are generated. Furthermore, no catalyst initiator or curing agents are necessary for the polymerization to take place. It is believed that the polymerization takes place when the cyclobutene ring undergoes transformation to prepare an aryl radical with two olefinic unsaturated moieties ortho to one another wherein the olefinic unsaturated moieties thereafter undergo reaction with the unsaturated cyclic imide moieties. It is to be noted that the temperature at which polymerization is initiated is dependent upon the nature of substituents on the cyclobutene ring. In general, wherein the cyclobutene ring is unsubstituted, the polymerization is initiated at 200°C. Wherein the cyclobutene ring is substituted with an electron-donating substituent, the polymerization temperature is generally lowered, the higher the ability of the substituent to donate electrons, the lower the polymerization initiation temperature is.

The method of polymerization of the N-substituted arylcyclobutenyl-maleimide monomers has a significant effect on the nature and properties of the polymeric composition prepared. In one embodiment, the N-substituted arylcyclobutenyl-maleimide monomers of this invention can be melt polymerized. The melt polymerization of N-substituted arylcyclobutenyl-maleimide monomers allows their use in the preparation of solid parts, as coatings, in composites, as adhesives and as fibers.

In one embodiment of the melt polymerization, the monomers are heated to the temperature at which it melts, preferably this is a temperature of between 80°C and 100°C, and thereafter poured or injected into a mold. Thereafter, pressure may be applied on the melted monomer in the mold. Generally, pressures of between atmospheric and 2000 psi (14 MPa) are suitable. Thereafter, the monomer is heated to a temperature at which the monomers undergo polymerization. This is preferably a temperature of between 200°C and 300°C, more preferably between 200°C and 250°C for between 10 minutes and 3 hours. Upon cooling, the polymerized composition can be removed from the mold.

Polymers prepared in this manner can subsequently be thermally treated at temperatures above 200°C to raise the modulus and lower the coefficient of expansion of such polymeric compositions.

In general, the polymers prepared by this method are insoluble in that they swell but do not dissolve, are thermally stable at 200°C, have a good modulus, a low water pickup and are reasonably hard.

In another embodiment, the N-substituted arylcyclobutenyl-maleimide monomers of this invention can be used to prepare coatings and films. In such embodiments, the monomers are dissolved in a suitable solvent and coated onto the substrate of choice, and thereafter the coated substrate is exposed to temperatures at which the monomers undergo polymerization over a period of time sufficient for the polymerization to go to completion. Under preferable conditions, temperatures of above 200°C for between 1 and 5 hours are used. Suitable solvents are those which volatilize away at temperatures below the polymerization temperature. Preferred solvents are cyclic and aliphatic ethers, lower alkanols, amides, and chlorinated hydrocarbon solvents. It is preferable to saturate the solvent with the monomer, a 20 to 30 weight percent concentration of monomer in the solvent is preferred.

The N-substituted arylcyclobutenyl-maleimide monomers may be combined with the powder-form or fibrous fillers or reinforcing materials either before or after heat treatment. For example, it is Possible to impregnate powder-form or fibrous fillers or reinforcing materials such as quartz sand or glass cloths, with the N-substituted arylcyclobutenyl-maleimide monomers, optionally in solution.

Suitable fillers and reinforcing materials are, generally, in any powder form and/or fibrous products, for example, of the type commonly used in the production of moldings based on unsaturated polyester resins or epoxide resins. Examples of products such as these are, primarily, granular fillers such as quartz powder,ground shale, asbestos powder, powdered corundum, chalk, iron powder, aluminum powder, sand, and gravel, also inorganic or organic fibers, more especially glass fibers in the usual textile forms of fibers, filaments rovings, yarns, nonwovens, mats and cloths. In this connection, amino silane-based finishes have proven to be particularly effective. It is also possible to use corresponding textile structures of organic, preferably synthetic fibers (polamides, polyesters) or on the basis of quartz, carbon, and metals, as well as monocrystals (whiskers).

The end products combined with fillers or reinforcing materials may be used in particular in vessel and pipe construction by the winding technique, in electrical engineering, in mold construction and tool making and also in the construction of heavily stressed components, in the lightweight construction of vehicles in aeronautical and astronautical engineering.

In another embodiment, the N-substituted arylcyclobutenyl-maleimide monomers can be used as adhesives. In such embodiment, one of the substrates to be joined is contacted with some form of the monomers, for example, the monomer in a powdered form. Thereafter, the second substrate to be adhesivated is contacted with the substrate previously contacted with the monomer where the monomer was contacted with the first substrate. Thereafter, pressure of at least 1 psi (7 KPa) is applied and the monomers and substrates are raised to a temperature at which the monomer undergoes polymerization.

In one embodiment, the N-substituted arylcyclobutenyl-maleimide monomers can be formed into a prepolymer which thereafter can be polymerized. To form the prepolymer, the N-substituted aryl-cyclobutenyl-maleimide monomers are contacted in an inert atmosphere or under vacuum and heated to a stage at which the polymerizaticn mixture is sufficiently viscous enough to be moldable in conventional molding equipment. In general, the mononers can be contacted at a temperature of 190°C to 220°C for between 1 and 10 minutes. Thereafter, the prepolymer can be used in various techniques to prepare the polymeric compositions of this invention. In one preferred embodiment, the prepolymer is cooled to form a powder which can be used to form compression molded articles, as an adhesive, and in many other uses. In another embodiment, a prepolymer of the N-substituted arylcyclobutenyl-maleimide monomers can be prepared by precipitation polymerization. In particular, the technique involves heating such monomers in a solvent to prepare a low molecular weight prepolymer that contains unreacted arylcyclobutene rings. A solvent is used which dissolves the monomer but not the prepolymer. As the prepolymer forms, it precipitates and is removed. The prepolymer can be fabricated in a hot compression mold which reacts out the remaining arylcyclobutene rings to give a thermoset polymer. The product is a fine white powder.

Preferable solvents are nonpolar solvents, such as aromatic hydrocarbons, aliphatic hydrocarbons, aliphatic chlorinated hydrocarbons, aromatic chlorinated hydrocarbon solvents, biphenols, naphthalenes or polychlorinated biphenols. The polymerization can take place at temperatures generally of between 200°C and 240°C for periods of between 1 and 5 hours. In general, the monomer can be dissolved up to saturation in the solvent used. A 20 to 30 percent by weight solution of the monomer in the solvent is preferred.

In another embodiment, the N-substituted arylcyclobutenyl-maleimide monomers can be polymerized by solution polymerization techniques. In this embodiment, the monomers are dissolved in dipolar aprotic solvents with boiling points above the polymerization temperature of the monomers. It is preferable that the solvents have a boiling point of above 200°C and more preferable that the solvents have a boiling point of above 250°C. Examples of preferred dipolar aprotic solvents include amides and sulfones. It is necessary to add to the solution lithium salts which solubilize the monomer in the solvents, preferably between 5 and 20 weight percent based on the monomer. A preferred lithium salt is lithium chloride. The polymerization takes place by heating the polymerization solution to a temperature at which the monomer undergoes polymerization, preferably above 200°C. The polymerization time is generally between 1 and 10 hours. The polymer can be recovered by adding water to precipitate the polymer from the reaction solution and thereafter stripping off the solvent. The polymers prepared with this method can be used in compression moldings or to prepare coatings. It is often desirable to process these polymers under elevated temperatures.

In another embodiment, the monomers of this invention which undergo polymerization at a temperature which is below the melting point of the monomer can be polymerized in a solid state polymerization. In this method, the monomers are heated to a temperature at which polymerization takes place. Polymers prepared in this method can be useful in the preparation of bearings, seals and other parts by powder metallurgy techniques.

The following examples are included to illustrate the invention, and do not limit the scope of the invention or the claims. Unless otherwise specified, all parts and percentages are by weight.

### Example 1 Preparation of 4-(N-Maleimido)phenyl-4-Benzocyclobutenyl Ketone

### (a) Preparation of 4-Nitrophenyl-4-Benzocyclobutenyl Ketone

Into a 100 ml roundbottom one neck flask equipped with a magnetic stirring bar, a reflux condenser and a nitrogen inlet was placed benzocyclobutene 10g (96.15 mmol) and 4-nitrobenzoyl chloride 11.9g (64.2mmol). To the flask was added Fe₂O₃ (0.65mmol, 1mol %). The flask was heated to 150°C under a nitrogen atmosphere with vigorous stirring overnight. The mixture was cooled to room temperature, mixed with chloroform (120 ml) and transferred to a separatory funnel. The dark brown solution was washed with 10 percent aqueous sodium bicarbonate (50 ml) twice, water (50 ml) and brine (50 ml) each once. The solution was dried over magnesium sulfate overnight and filtered through celite. The volatiles were removed on a rotovap to give a deep red-black viscous liquid. The liquid was contacted with 100 ml of n-hexane and heated to boiling. The hot n-hexane was decanted away from the undissolved brown liquid. The hexane treatment was repeated three more times. The resultant yellow n-hexane layers were combined and slowly cooled to room temperature. An off-white solid (rosettes) precipitated out of solution. The solid was isolated by suction filtration. The filtrate was concentrated to one half of its volume and allowed to stand. The solid recovered has a weight of 4.8 grams (a 30 percent yield). NMR and IR spectra were taken of the solid and the spectra agreed with the structure of 4-nitrophenyl-4-benzocyclobutenyl ketone.

### (b) Preparation of 4-Aminophenyl-4-Benzocyclobutenyl Ketone

Into a 250 ml three neck round bottom flask equipped with a magnetic stirring bar, a reflux condensor with a nitrogen inlet and a thermometer and an equilibrating addition funnel was charged 1.0 g (3.95 mmol) of 4-nitrophenyl-4-benzocyclobutenyl ketone, 4.46 g (19.75 mmol) of (SnCl₂·2H₂O) and 100 ml of ethanol. Under a nitrogen atmosphere, the mixture was heated to 60°C. To the mixture, in a slow dropwise manner was added sodium borohydride, 75 mg (1.975 mmol) in 20 ml of ethanol, over a period of 20 minutes. After the addition, the temperature of the mixture was maintained at 60°C for 30 minutes. The mixture was cooled to 10°C and 80ml of water previously chilled was added. Concentrated sodium hydroxide was added until the pH is 7 (4 M, NaOH, about 6 ml). The mixture was transferred to a 500 ml round-bottom flask and the ethanol was removed on a rotovap. Thereafter, 100 ml of water was added to the off-while slurry and the aqueous phase was extracted with diethyl ether four times with 100 ml aliquots. The ether extracts were dried over sodium sulfate overnight. The sodium sulfate was filtered from the diethyl ether extracts and the diethyl ether was removed on a rotovap. A bright orange solid (0.87g) was obtained. The solid was recrystallized using carbon tetrachloride (200 ml) and decolorizing charcoal. The first crop of crystals was a pale yellow solid 0.54 g (61.3%). The carbon tetrachloride was concentrated to 50 ml and left standing overnight. NMR and IR indicated the product iss 4-aminophenyl-4-benzocyclobutenyl ketone.

### (c) Preparation of 4-(N-Maleimido)phenyl-4-Benzocyclobutenyl Ketone

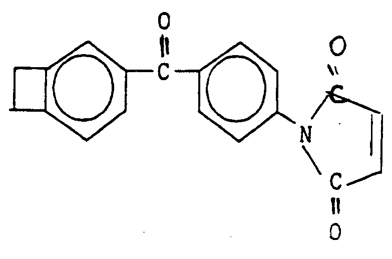

Into a 100 ml round bottom flask equipped with a magnetic stirring bar and a nitrogen inlet was placed 1.0g(4.482 mmol) of 4-aminophenyl-4-benzocyclobutenyl ketone and 20 ml of acetone. To the solution is added 4.40g(4.482 mmol) of maleic anhydride in several portions over two minutes, with stirring. The solution was stirred at room temperature for two days. A white solid precipitated. The white solid is believed to be 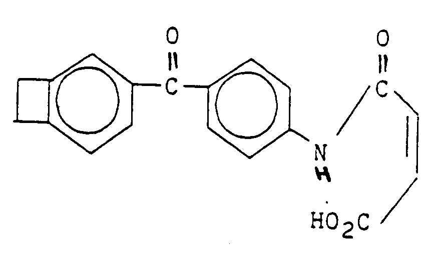
The white solid and the solution described above were stirred with 4.9mg(8.955 mmol,0 .85ml) of acetic anhydride, 35 mg(0.142 mmol) of hydrated sodium acetate (four waters of hydration) and 19 drops of triethyl amine. As the triethyl amine was added, the solution turned yellow. Stirring at room temperature was continued overnight. The reaction mixture was an orange hazy mixture (fine solid was present). The reaction mixture was poured into 80 ml of vigorously stirred aqueous sodium bicarbonate. An orange solid precipitated from the solution. To the solution was added 100 ml of chloroform, and the solution was transferred to a separatory funnel. The layers were separated and the aqueous phase was extracted with 100 ml of chloroform. The chloroform extracts were combined and washed with 50 ml of water, and then 50 ml of brine. The solution was dried over magnesium sulfate and the suction filtered through celite. The solvent was removed by a vacuum to give 1.20g of an orange viscous syrup. The product was recrystalized using ethanol and decolorizlng charcoal. The mixture was gravity filtered to give a pale yellow solution. The solution was concentrated to one-half its volume and placed in ice. A white solid precipitated. The solution was allowed to stand overnight. The solid was isolated and dried in air for 1 hour. The weight of the product was 0.49g, and has a melting point of 138-139°C. Another 0.39g of product was further isolated to give a total yield of 0.88g. The product was examined by IR and NMR and shows agreement with the following structure, 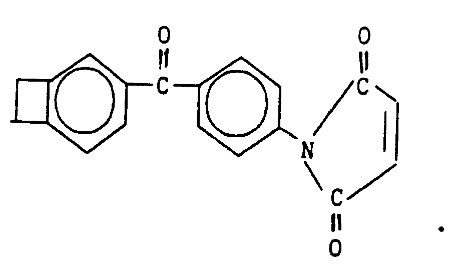
The DSC of the product indicated a melting point of 147°C and an exotherm was observed at 260.4°C. The energy of the exotherm is 347 J/g. A rescan of the DSC showed no melting point or exotherm but did exhibit a T₂ at 260.4°C.

### Example 2 - Polymerization of 4-(N-maleimido)phenyl-4-Benzocyclobutenyl Ketone

Into a tube was placed 146 mg of 4-(N-maleimido)-phenyl-4-benzocyclobutenyl ketone and nitrogen. The tube was placed into a Wood's Metal bath at 150°C. The temperature was controlled and monitored with a heater and a thermocouple. The following sequence of times and temperatures were applied to the tube:
150°C 30 min.
180°C 30 min.
210°C 30 min.
235°C 1 hour
260°C 1 hour
270°C 1 hour
At the end of the sequence, the heating was stopped and the tube and thermocouple were removed from the heating bath. The tube was allowed to cool overnight. The polymer was an amber color with small voids trapped in the matrix. The polymer was physically broken into small pieces. A TGA was run on one of the pieces, 0.05 wt % loss occurred at 327.78°C and 5% weight loss at 464.39°C.

## Claims

1. An N-substituted arylclobutenyl-maleimide which corresponds to the Formula I: 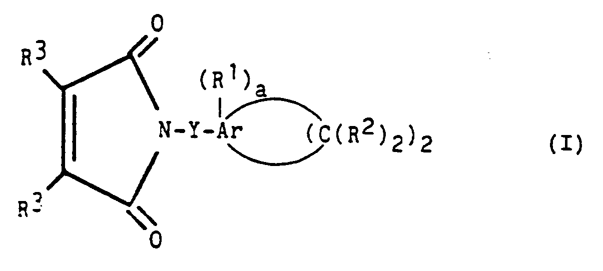 wherein
Ar is an aromatic radical;
R¹ is separately in each occurrence C₁₋₂₀ alkyl, C₁₋₂₀ alkoxy, C₁₋₂₀ alkythio, C₆₋₂₀ aryl, C₆₋₂₀ aryloxy, C₆₋₂₀ arylthio, C₇₋₂₀ alkaryl, C₇₋₂₀ alkaryloxy, C₇₋₂₀ alkarylthio, C₇₋₂₀ aralkyl, C₇₋₂₀ aralkoxy, C₇₋₂₀ aralkylthio, cyano, carboxylate, hydrocarbylcarbonyloxy, nitro, halo, hydrocarbylsulfinyl, hydrocarbylsulfonyl or amino;
R² is separately in each occurrence hydrogen, cyano, carboxylate, hydrocarbylcarbonyloxy, nitro, halo, hydrocarbylsulfonyl, hydrocarbylsulfinyl, alkyl, amido or hydrocarbyloxy;
R³ is separately in each occurrence hydrogen, C₁₋₂₀ alkyl, C₁₋₂₀ alkoxy, C₁₋₂₀ alkylthio, C₆₋₂₀ aryl, C₆₋₂₀ aryloxy, C₆₋₂₀ arylthio, C₇₋₂₀ alkaryl, C₇₋₂₀ alkaryloxy, C₇₋₂₀ alkarylthio, C₇₋₂₀ aralkyl, C₇₋₂₀ aralkoxy or C₇₋₂₀ aralkylthio;
Y is a divalent hydrocarbylene, hydrocarbylenecarbonyloxy, hydrocarbyleneoxy, hydrocarbyleneamino, hydrocarbylenecarbonyl, hydrocarbylenethio, hydrocarbylenepolythio, hydrocarbylenesulfinyl or hydrocarbylenesulfonyl;
and
a is an integer of between 0 and 3.

2. A compound as claimed in Claim 1, which corresponds to the Formula II: 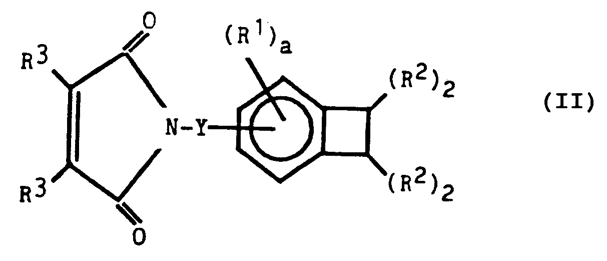 wherein
R¹, R², R³, Y and a are as defined in Claim 1.

3. A compound as claimed in Claim 2, wherein R¹ is separately in each occurrence C₁-C₂₀ alkyl, halogen, nitro or cyano; R² is separately in each occurrence hydrogen, halogen, nitro or cyano; and R³ is separately in each occurrence hydrogen or C₁-C₂₀ alkyl.

4. A compound as claimed in any one of the preceding claims, wherein Y is hydrocarbylenecarbonyl.

5. A compound as claimed in Claim 4, wherein Y is p-phenylenecarbonyl.

6. A compound as claimed in any one of the preceding claims, wherein R² and R³ are all hydrogen and a is 0.

7. 4-(N-Maleimido)phenyl-4-Benzocyclobutenyl Ketone.

8. A polymeric composition comprising a polymer of a compound as claimed in any one of the preceding claims.

9. A polymeric composition as claimed in Claim 8, which corresponds to the formula: 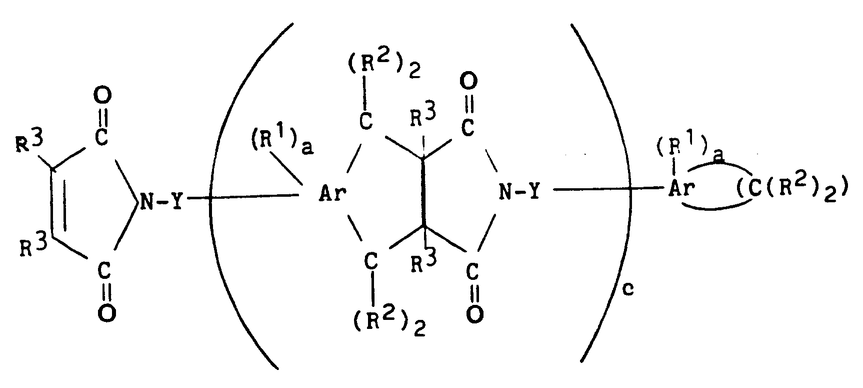 wherein
Ar, R¹, R², R³, Y and a are as defined in Claim 1 and
c is a real number of 2 or greater.

10. A polymer composition as claimed in Claim 8 which corresponds to the formula: 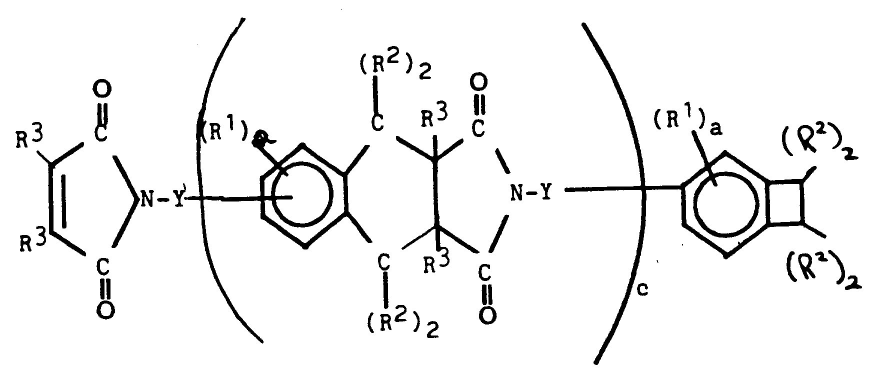 wherein
R¹, R², R³, Y and a are as defined in Claim 2 and
c is an real number of 2 or greater.

11. A polymeric composition as claimed in Claim 9 or Claim 10, wherein R¹ is separately in each occurrence C₁₋C₂₀ alkyl, halogen, nitro or cyano; R² is separately in each occurrence hydrogen, halogen, nitro or cyano; and R³ is separately in each occurrence hydrogen or C₁-C₂₀ alkyl.

12. A polymeric composition as claimed in any one of Claims 9 to 11, wherein Y is hydrocarbylenecarbonyl.

13. A polymeric composition as claimed in Claim 12, wherein Y is p-phenylenecarbonyl.

14. A polymeric composition as claimed in any one of Claims 9 to 13, wherein R² and R³ are all hydrogen and a is 0.

15. A polymer of 4-(N-Maleimido)phenyl-4-Benzocyclobutenyl Ketone.

16. A method of preparing a polymer as claimed in any one of Claims 8 to 15 which comprises heating an N-substituted arylcyclobutenylmaleimide as claimed in Claim 1 to a temperature above 170°C.

17. A method as claimed in Claim 16, wherein the N-substituted arylcyclobutenyl-maleimide is as claimed in any one of Claims 2 to 7.

18. A method as claimed in Claim 16 or Claim 17, wherein the N-arylcyclobutenyl-maleimide is heated in the absence of a catalyst initiator or curing agent.

19. A method of preparing an N-arylcyclobutenyl-maleimide as claimed in any one of Claims 1 to 7 which comprises
(a) when Y is hydrocarbyleneoxycarbonyl, reacting a corresponding N-hydroxyhydrocarbyl maleimide with a corresponding chlorocarbonyl-substituted arylcyclobutene in the presence of a tertiary amine;
(b) when Y is hydrocarbyleneoxy, reacting a corresponding maleimido hydrocarbyl p-toluene sulfonate with a corresponding hydroxy-substituted arylcyclobutene in the presence of a 4 to 5 molar excess of an alkali metal carbonate in the presence of a N,N-dimethylformamide solvent;
(c) when Y is hydrocarbyleneamino, dehydrating a corresponding hydrocarbyleneamino-bridged N-arylcyclobutenyl amido alkenoic acid by contact with a dehydrating agent in an aprotic reaction medium in the presence of a nickel (II) salt catalyst or by heating in a glacial acetic acid medium in the presence of an alkali or alkaline earth metal acetate salt;
(d) when Y is hydrocarbylene, dehydrating a corresponding N-hydrocarbylarylcyclobutene amido alkenoic acid by contact with a dehydrating agent in an aprotic reaction medium in the presence of a nickel (II) salt catalyst or by heating in a glacial acetic acid medium in the presence of an alkali or alkaline earth metal acetate salt;
(e) when Y is hydrocarbylenethio, dehydrating a corresponding hydrocarbylenethio-bridged N-arylcyclobutenyl amido alkenoic acid by contact with a dehydrating agent in an aprotic reaction medium in the presence of a nickel (II) salt catalyst or by heating in a glacial acetic acid medium in the presence of an alkali or alkaline earth metal acetate salt;
(f) when Y is hydrocarbylenesulfinyl, contacting a corresponding hydrocarbylenethio-bridged N-arylcyclobutenyl maleimide with an equimolar amount of peracetic acid in an ethyl acetate solvent at 0°C to 20°C; and
(g) when Y is hydrocarbylenesulfonyl, contacting a corresponding hydrocarbylenethio-bridged N-arylcyclobutenyl maleimide with about 2 moles of peracetic acid in an ethyl acetate solvent at 0°C to 20°C.

## Patentansprüche

1. N-substituierte Arylcyclobutenylmaleinimide der Formel I: 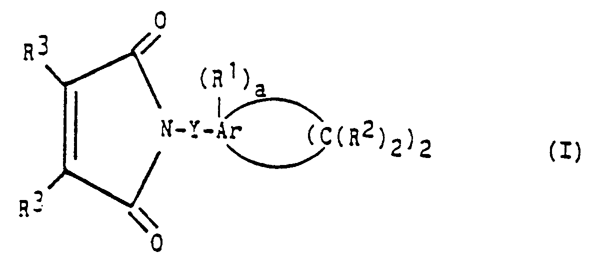 in der
Ar einen aromatischen Rest bezeichnet;
R¹ jeweils unabhängig bei jedem Vorkommen C₁₋₂₀-Alkyl, C₁₋₂₀-Alkoxy, C₁₋₂₀-Alkylthio, C₆₋₂₀-Aryl, C₆₋₂₀-Aryloxy, C₆₋₂₀-Arylthio, C₇₋₂₀-Alkaryl, C₇₋₂₀-Alkaryloxy, C₇₋₂₀-Alkarylthio, C₇₋₂₀-Aralkyl, C₇₋₂₀-Aralkoxy, C₇₋₂₀-Aralkylthio, Cyano, Carboxylat, Hydrocarbylcarbonyloxy, Nitro, Halogen, Hydrocarbylsulfinyl, Hydrocarbylsulfonyl oder Amino bezeichnet;
R² jeweils unabhängig bei jedem Vorkommen für Wasserstoff, Cyano, Carboxylat, Hydrocarbylcarbonyloxy, Nitro, Halogen, Hydrocarbylsulfonyl, Hydrocarbylsulfinyl, Alkyl, Amido oder Hydrocarbyloxy steht;
R³ jeweils unabhängig bei jedem Vorkommen Wasserstoff, C₁₋₂₀-Alkyl, C₁₋₂₀-Alkoxy, C₁₋₂₀-Alkylthio, C₆₋₂₀-Aryl, C₆₋₂₀-Aryloxy, C₆₋₂₀-Arylthio, C₇₋₂₀-Alkaryl, C₇₋₂₀-Alkaryloxy, C₇₋₂₀-Alkarylthio, C₇₋₂₀-Aralkyl, C₇₋₂₀-Aralkoxy oder C₇₋₂₀-Aralkylthio bezeichnet;
Y für einen zweiwertigen Hydrocarbylen-, Hydrocarbylencarbonyloxy, Hydrocarbylenoxy-, Hydrocarbylenamino-, Hydrocarbylencarbonyl-, Hydrocarbylenthio-, Hydrocarbylenpolythio-, Hydrocarbylensulfinyl- oder Hydrocarbylensulfonyl-Rest steht; und
a eine ganze Zahl (integer) von zwischen 0 und 3 bedeutet.

2. Verbindungen nach Anspruch 1, die der Formel II: 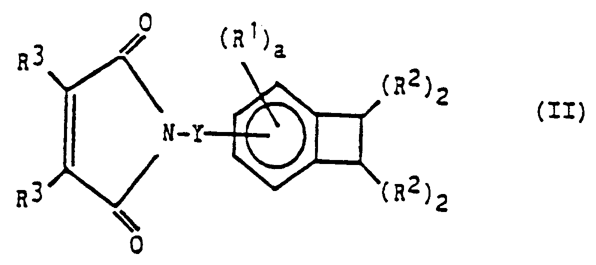 entsprechen, in der R¹ , R² , R³ , Y und a die in Anspruch 1 angegebene Bedeutung haben.

3. Verbindungen nach Anspruch 2, wobei R¹ jeweils unabhängig bei jedem Vorkommen C₁₋₂₀-Alkyl, Halogen, Nitro oder Cyano; R² jeweils unabhängig bei jedem Vorkommen Wasserstoff, Halogen, Nitro oder Cyano; und R³ jeweils unabhängig bei jedem Vorkommen Wasserstoff oder C₁₋₂₀-Alkyl bedeutet.

4. Verbindungen nach jedem der Vorhergehenden Ansprüche, in denen Y Hydrocarbylencarbonyl bedeutet.

5. Verbindungen nach Anspruch 4, in denen Y für para-Phenylencarbonyl steht.

6. Verbindungen nach jedem der Vorhergehenden Ansprüche, wobei alle Substituenten R² und R³ Wasserstoff sind und a gleich 0 ist.

7. 4-(N-Maleinimido)phenyl-4-benzocylobutenylketon.

8. Polymerengemisch, enthaltend ein Polymeres einer Verbindung nach irgendeinem der vorhergehenden Ansprüche.

9. Polymerengemisch nach Anspruch 8, entsprechend der Formel 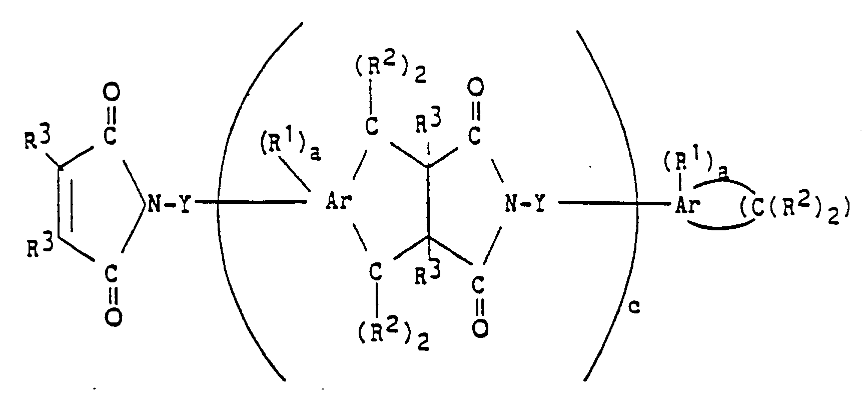 in der Ar, R¹ , R² , R³ , Y und a die in Anspruch 1 angegebene Bedeutung haben und
c für eine reale Zahl (real number) von 2 oder größer steht.

10. Polymerengemisch nach Anspruch 8, entsprechend der Formel: 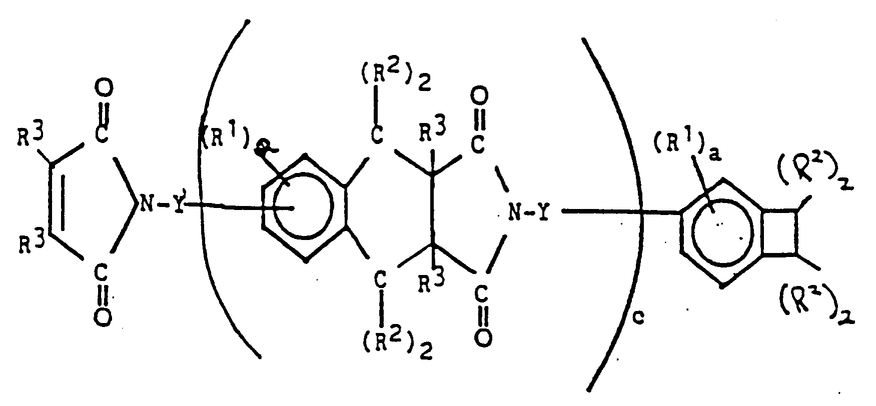 in der R¹, R², R³, Y und a die in Anspruch 2 angegebene Bedeutung haben und
c für eine reale Zahl von 2 oder größer steht.

11. Polymerengemisch nach Anspruch 9 oder 10, wobei R¹ jeweils unabhängig bei jedem Vorkommen C₁₋₂₀-Alkyl, Halogen, Nitro oder Cyano; R² jeweils unabhängig bei jedem Vorkommen Wasserstoff, Halogen, Nitro oder Cyano; und R³ jeweils unabhängig bei jedem Vorkommen Wasserstoff oder C₁₋₂₀-Alkyl bedeutet.

12. Polymerengemisch nach jedem der Ansprüche 9 bis 11, wobei Y Hydrocarbylencarbonyl bedeutet.

13. Polymerengemisch nach Anspruch 12, wobei Y para-Phenylencarbonyl bezeichnet.

14. Polymerengemisch nach jedem der Ansprüche 9 bis 13, wobei alle Substituenten R² und R³ Wasserstoff sind und a gleich 0 ist.

15. Polymeres aus 4-(N-Maleinimido)phenyl-4-benzocyclobutenylketon.

16. Verfahren zur Herstellung der Polymeren nach jedem der Ansprüche 8 bis 15, wobei man ein N-substituiertes Arylcyclobutenylmaleinimid nach Anspruch 1 auf eine Temperatur oberhalb von 170°C erhitzt.

17. Verfahren nach Anspruch 16, wobei das N-substituierte Arylcyclobutenylmaleinimid ein solches ist, wie es in jedem der Ansprüche 2 bis 7 beansprucht wurde.

18. Verfahren nach Anspruch 16 oder 17, wobei das N-Arylcyclobutenylmaleinimid in Abwesenheit eines initiierenden Katalysators oder eines Vernetzungs- oder Härtungsmittels erhitzt wird.

19. Verfahren zur Herstellung von N-Arylcyclobutenylmaleinimiden nach jedem der Ansprüche 1 bis 7, wobei man,
(a) sofern Y einen Hydrocarbylenoxycarbonyl-Rest bedeutet, ein entsprechendes N-Hydroxyhydrocarbylmaleinimid mit einem entsprechenden Chlorcarbonyl-substituierten Arylcyclobuten in Gegenwart eines tertiären Amins umsetzt;
(b) sofern Y einen Hydrocarbylenoxy Rest-bedeutet, ein entsprechendes Maleinimidohydrocarbyl-para-toluolsulfonat mit einem entsprechenden Hydroxy-substituierten Arylcyclobuten in Gegenwart eines 4- bis 5-fachen Überschusses eines Alkalimetallcarbonates und in Gegenwart von N,N-Dimethylformamid als Lösungsmittel umsetzt;
(c) sofern Y einen Hydrocarbylenamino-Rest bedeutet, eine N-Arylcyclobutenylamidoalkensäure mit entsprechender Hydrocarbylenamino-Brücke dehydratisiert, indem man sie in einem aprotischen Medium und in Gegenwart eines Nickel (II)-Salzes als Katalysator mit einem Dehydratisierungsmittel in Berührung bringt oder indem man sie in Eisessig in Gegenwart eines Alkali- oder Erdalkaliacetats erhitzt;
(d) sofern Y einen Hydrocarbylen-Rest bedeutet, eine entsprechende N-Hydrocarbylarylcyclobutenamidoalkensäure durch Kontakt mit einem Dehydratisierungsmittel in einem aprotischen Lösungsmittel und in Gegenwart eines Nickel (II)-Salzes oder durch Erhitzen in Eisessig in Gegenwart eines Alkali- oder Erdalkaliacetats dehydratisiert;
(e) sofern Y einen Hydrocarbylenthio-Rest bedeutet, eine entsprechende N-Arylcyclobutenylamidoalkensäure mit Hydrocarbylenthio-Brücke durch Kontakt mit einem Dehydratisierungsmittel in einem aprotischen Lösungsmittel und in Gegenwart eines Nickel(II)-Salzes als Katalysator oder durch Erhitzen in Eisessig in Gegenwart eines Alkali- oder Erdalkaliacetats dehydratisiert;
(f) sofern Y einen Hydrocarbylensulfinyl-Rest bedeutet, ein entsprechendes N-Arylcyclobutenylmaleinimid mit Hydrocarbylenthio-Brücke mit einer äquimolaren Menge Peressigsäure in Ethylacetat als Lösungsmittel bei 0°C bis 20°C in Berührung bringt; und
(g) sofern Y einen Hydrocarbylensulfonyl-Rest bedeutet, ein entsprechendes N-Arylcyclobutenylmaleinimid mit Hydrocarbylenthio-Brücke mit etwa 2 Molen Peressigsäure in Ethylacetat als Lösungsmittel bei 0°C bis 20°C in Berührung bringt.

## Revendications

1. Un arylcyclobutényl-maléimide N-substitué correspondant à la formule I : 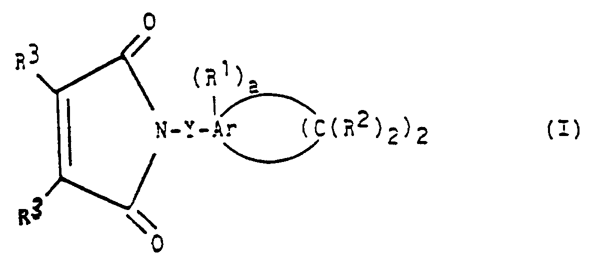 dans laquelle
Ar est un radical aromatique;
R¹ est, indépendamment dans chaque cas, alkyle en C₁₋₂₀, alcoxy en C₁₋₂₀, alkylthio en C₁₋₂₀, aryle en C₆₋₂₀, aryloxy en C₆₋₂₀, arylthio en C₆₋₂₀, alkaryle en C₇₋₂₀, alkaryloxy en C₇₋₂₀, alkarylthio en C₇₋₂₀, aralkyle en C₇₋₂₀, aralcoxy en C₇₋₂₀, aralkylthio en C₇₋₂₀, cyano, carboxylate, hydrocarbylcarbonyl-oxy, nitro, halogène, hydrocarbylsufinyl, hydrocarbylsulfonyl, ou amino;
R² est, indèpendamment dans chaque cas, hydrogène, cyano, carboxylate, hydrocarbylcarbonyl-oxy, nitro, halogène, hydrocarbylsulfonyle, hydrocarbylsulfinyle, alkyle, amido ou hydrocarbyloxy;
R³ est, indépendamment dans chaque cas, hydrogène, alkyle en C₁₋₂₀; alcoxy en C₁₋₂₀, alkylthio en C₁₋₂₀, aryle en C₆₋₂₀, aryloxy en C₆₋₂₀, arylthio en C₆₋₂₀, alkaryle en C₇₋₂₀, alkaryloxy en C₇₋₂₀, alkarylthio C₇₋₂₀ aralkyle en C₇₋₂₀, aralcoxy en C₇₋₂₀ ou C₇₋₂₀ aralkylthio;
Y est un groupe divalent hydrocarbylène, hydrocarbylènecarbonyloxy, hydrocarbylène-oxy, hydrocarbylène-amino, hydrocarbylène-carbonyle, hydrocarbylène-thio, hydrocarbylène-polythio, hydrocarbylène-sulfinyl ou hydrocarbylène-sulfonyl;
et
a est un entier valant de 0 à 3.

2. Composé selon la revendication 1, correspondant à la formule II : 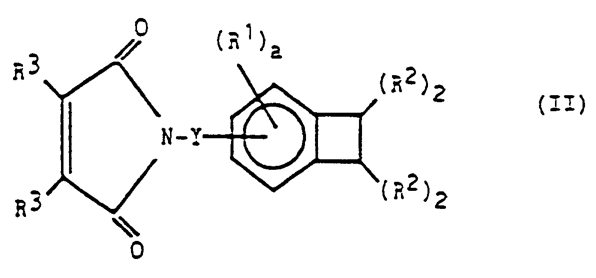 dans laquelle
R1, R², R³, Y et a sont tels que définis à la revendication 1.

3. Composé selon la revendication 2, dans lequel R¹ est, indépendamment dans chaque cas alkyle en C₁₋₂₀, halogène, nitro ou cyano; R² est, indépendamment dans chaque cas, hydrogène, halogène, nitro ou cyano; et R³ est indépendamment dans chaque cas, hydrogène ou alkyle en C₁-C₂₀.

4. Composé selon l'une quelconque des revendications précédentes, dans laquelle Y est hydrocarbylène-carbonyle.

5. Composé selon la revendication 4, dans lequelle Y est p-phénylène-carbonyle.

6. Composé tel que défini à l'une quelconque des revendications précédentes, dans lequel R² et R³ sont tous un hydrogène et a est 0.

7. 4-(N-maléimido)phényl-4-benzocyclobutènyle-cétone.

8. Composition polymérique comprenant un polymère d'un composé selon l'une quelconque des revendications précédentes.

9. Composition polymérique selon la revendication 8, corres pondant à la formule : 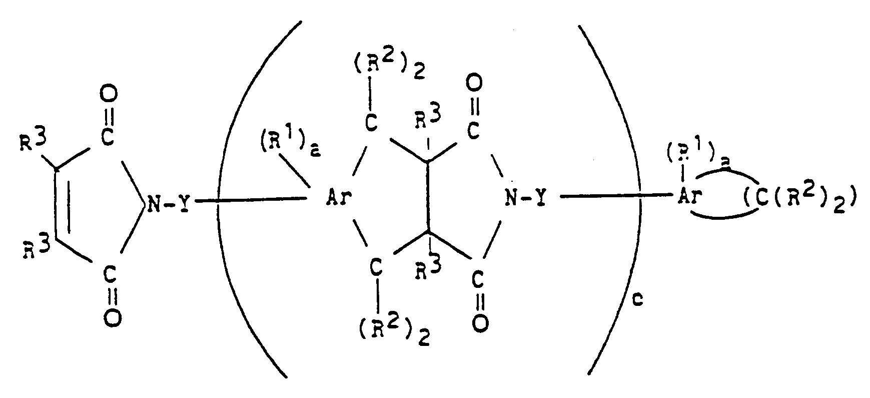 dans laquelle
Ar, R¹, R², R³, Y et a sont définis à la revendication 1 et
c est un nombre réel égal à 2 ou plus grand.

10. Composition polymérique selon la revendication 8 correspondant à la formule : 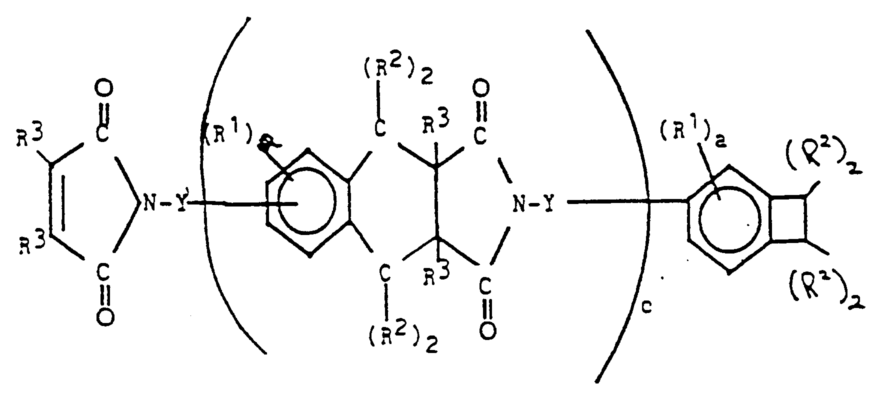 dans laquelle
R¹, R², R³, Y e a sont définis à la revendication 2 et
c est un un nombre réel égal à 2 ou plus grand.

11. Composition polymérique selon la revendication 9 ou la revendication 10, dans laquel R¹ est indépendamment dans chaque cas, alkyle en C₁-C₂₀, halogène, nitro ou cyano; R² est indépendamment dans chaque cas, hydrogène, halogène, nitro ou cyano; et R³ est, indépendamment dans chaque cas hydrogène ou alkyle en C₁₋₂₀.

12. Composition polymérique selon l'une quelconque des revendications 9 à 11, dans laquelle Y est hydrocarbylène-carbonyle.

13. Composition polymérique selon la revendication 12 dans laquelle Y est p-phénylène-carbonyle.

14. Composition polymérique selon l'une des revendications 9 à 13, dans laquelle R² et R³ sont l'hydrogène et a vaut 0.

15. Polymère de 4-(maléimido)phényl-4-benzocyclobutènyl-cétone.

16. Procédé de préparation d'un polymère tel que défini dans l'une des revendications 8 à 15, comportant le chauffage d'un arylcyclobutényl-maléimide N-substitué tel que défini à la revendication 1 à une température supérieure à 170°C.

17. Procédé selon la revendication 16 dans laquelle l'arylcyclobutényl-maléimide N-substitué est tel que défini dans l'une quelconque des revendications 2 à 7.

18. Procédé selon la revendication 16 ou la revendication 17, dans lequel le N-arylcyclobutényl-maléimide est chauffé en absence de catalyseur initiateur ou d'agent de polymérisation.

19. Procédé de préparation d'un N-asylcyclobutényl-maléimide tel que défini dans l'une quelconque des revendications 1 à 7 comportant
(a) lorsque Y est un hydrocarbylène-oxycarbonyle, la réaction de la N-hydroxyhydrocarbyl-maléimide correspondant avec l'arylcyclobutène à substituant chlorocarbyle correspondant en présence d'une amine tertiaire;
(b) lorsque Y est un hydrocarbylène-oxy, la réaction du paratoluène-sulfonate de maléimido-hydrocarbyle correspondant avec l'arylcyclobutène hydroxy-substitué correspondant en présence d'un excès molaire de 4 à 5 fois d'un carbonate de métal alcalin et en présence du solvant N,N-diméthylformamide;
(c) lorsque Y est hydrocarbylène-amino, la déshydratation de l'acide N-arylcyclobuténylamido-alcénoïque à pont hydrocarbylèneamino par contact avec un agent déshydratant dans un milieu réactionnel aprotique en présence d'un catalyseur constitué de sel de nickel (II) ou par chauffage dans un milieu d'acide acétique glacial en présence d'un acétate de métal alcalin ou alcalino-terreux;
(d) lorsque Y est un hydrocarbylène, en déshydratant l'acide N-hydrocarbylarylcyclobutène-amido alkénoïque correspondant par contact avec un agent déshydratant dans un milieu réactionnel aprotique en présence d'un catalyseur constitué de sel de nickel (II) ou par chauffage dans un milieu à l'acide acétique glacial en présence d'un acétate de métal alcalin ou alcalino-terreux;
(e) quand Y est hydrocarbylène-thio, en déshydratant l'acide N-arylcyclobutényl-amido alkénoïque à pont hydrocarbylène-thio par contact avec un agent déshydratant dans un milieu réactionnel aprotique en présence d'un catalyseur constitué de sel de nickel (II) ou par chauffage dans un milieu à l'acide acétique glacial en présence d'un acétate de métal alcalin ou alcalino-terreux;
(f) quand Y est hydrocarbylène-sulfinyle, par mise en contact du N-arylcyclobuténylmaléïmide à pont hydrocarbylène-thio correspondant avec un équivalent molaire d'acide peracétique dans le solvant acétate d'éthyle entre 0°C et 20°C; et
(g) quand Y est hydrocarbylène-sulfonyle, par mise en contact du N-arylcyclobuténylmaléïmide à pont hydrocarbylène-thio correspondant avec environ 2 moles d'acide acétique dans le solvant acétate d'éthyle entre 0°C et 20°C.
